# EUROPEAN PATENT APPLICATION

(11) **EP 4 624 474 A1**
(43) Date of publication of application: **01.10.2025**
(21) Application number: 23893960.7
(22) Date of filing: 23.11.2023
(51) Int. Cl.: C07D 519/00, C07D 471/04, A61K 31/4375, C07D 401/12, A61P 35/00

(54) **PHARMACEUTICALLY ACCEPTABLE SALT OF NITROGEN-CONTAINING HETEROCYCLIC COMPOUND, CRYSTAL FORM THEREOF, AND PREPARATION METHOD THEREFOR**

(30) Priority: 23.11.2022 CN 202211471375
(71) Applicant: Jiangsu Hengrui Pharmaceuticals Co., Ltd., Lianyungang, Jiangsu 222047 (CN); Shanghai Hengrui Pharmaceutical Co., Ltd., Shanghai 200245 (CN)
(72) Inventor: SHANG, Tingting, Lianyungang, Jiangsu 222047 (CN); YANG, Junran, Lianyungang, Jiangsu 222047 (CN); DU, Zhenxing, Lianyungang, Jiangsu 222047 (CN); SHAO, Cheng, Shanghai 200245 (CN); YOU, Lingfeng, Shanghai 200245 (CN)
(74) Representative: Dragotti & Associati S.R.L.
(86) International application number: PCT/CN2023/133568
(87) International publication number: WO 2024/109871

(57) **Abstract**

The present disclosure relates to a pharmaceutically acceptable salt of a nitrogen-containing heterocyclic compound, a crystal form thereof, and a preparation method therefor. Specifically, the present disclosure provides a pharmaceutically acceptable salt of a formula 1 compound (R)-3-((7-ethyl-6-oxo-5,6-dihydro-1,5-naphthyridine-3-yl)methyl)-N-methyl-1,2,3,4,4a,5-hexahydro-7H-pyrazino[2,1-c]pyrido[3,2-e][1,4]oxazepine-9-formamide, and a crystal form thereof, and a preparation method therefor, and the corresponding salt has good stability and can be better utilized in clinical treatment.

## Description

### TECHNICAL FIELD

The present disclosure pertains to the field of pharmaceutics and relates to a pharmaceutically acceptable salt and a crystal form of a nitrogen-containing heterocyclic compound and a preparation method therefor.

### BACKGROUND

Poly(ADP-ribose) polymerase 1 (PARP1) was first reported over 50 years ago, and was gradually found to play an important role in DNA repair, maintenance of genomic integrity, regulation of various metabolic and signal transduction processes, and the like. PARP1 is capable of catalyzing the transfer of ADP-ribose residues from NAD+ to a target substrate, thereby constructing a poly(ADP-ribose) (PAR) chain. The formation and clearance of the PAR chain occur in almost all eukaryotic cells.

ADP-ribosylation is a post-translational modification of proteins that is widely present in various physiological and pathological processes, and refers to the binding of one or more ADP-ribose units to specific sites of proteins by catalysis with enzymes. PARP1 is the first member of the PARP superfamily, which consists of proteins having homology to PARP1. There are 17 members, of which 4 (PARP1, PARP2, PARP5A, and PARP5B) are capable of synthesizing the PAR chain. Most other enzymes in the family can only construct a single ADP-ribose unit and are therefore classified as mono(ADP-ribosyl)ases (MARs).

PARP1 and PARP2 have been extensively studied for their roles in DNA damage repair. PARP1 is activated by DNA damage and functions to catalyze the formation of a poly(ADP-ribose) (PAR) chain on a target protein. This post-translational modification, referred to as poly-ADP-ribosylation (PARylation), can mediate the recruitment of other DNA repair factors to the DNA damage. Upon completion of this recruitment task, PARP auto-PARylation triggers the release of bound PARP from DNA, thereby allowing access to other DNA repair proteins to complete the repair. Therefore, the binding of PARP to damaged sites, its catalytic activity, and final release from DNA are all important steps in the response of cancer cells to DNA damage caused by chemotherapeutic agents and radiotherapy.

The inhibition of PARP family enzymes has been used as a strategy to selectively kill cancer cells by inactivating complementary DNA repair pathways. Numerous preclinical and clinical studies have shown that tumor cells with harmful changes of the key tumor suppressor protein BRCA1 or BRCA2 involved in the double-strand DNA break (DSB) repair through harmful recombination (HR) are selectively sensitive to small molecules. DNA repair enzymes and PARP family inhibitors. The homologous recombination repair (HRR) pathway of such tumors is deficient and depends on the survival function of PARP enzymes. Although PARP inhibitor therapy is mainly directed against BRCA-mutated cancers, PARP inhibitors have been clinically tested in non-BRCA-mutated tumors that exhibit homologous recombination deficiency (HRD).

PARP inhibitors with increased selectivity for PARP1 may have improved efficacy and reduced toxicity compared to other PARP1/2 inhibitors. We believe that a selective and strong inhibition of PARP1 will lead to the capture of PARP1 on DNA, resulting in DNA double-strand breaks (DSBs) by the collapse of replication forks in the S phase. PARP1-DNA capture is an effective mechanism to selectively kill tumor cells with HRD. Therefore, there is an urgent clinical need for effective and safe PARP inhibitors, particularly a PARP inhibitor with the selectivity for PARP1.

Related patent applications that have been disclosed include WO2021013735A1, WO2021260092A1, WO2009053373A1, WO2008107478A1, and the like. PCT/CN2022/094612 discloses a novel class of tetralin derivatives. Among these, the one represented by formula 1 has the chemical name (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, and its use as a PARP inhibitor is demonstrated. PCT/CN2022/094612 is incorporated in the present disclosure by reference in its entirety.

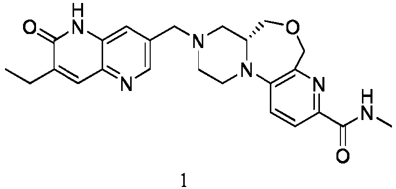

The structure of a crystal form used as a pharmaceutically active ingredient often affects the chemical and physical stabilities of the drug. Changes in crystallization and storage conditions may cause changes in the crystal structure of the compound and sometimes the generation of other crystal forms. Generally, drug products of amorphous forms do not have a regular crystal structure and often have other defects, such as having relatively poor product stability, being relatively difficult to filter, being prone to agglomeration, and having poor flowability. Therefore, research on pharmaceutically acceptable salts and crystal forms of the compound of formula 1, (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, is of great significance for the development of drugs that are suitable for industrial production and have good bioactivity.

### SUMMARY

In one aspect, the present disclosure provides a crystal form A of the compound of formula 1, (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide. In some embodiments, an X-ray powder diffraction pattern expressed in terms of 2*θ* angles, which are diffraction angles, of the crystal form A has characteristic peaks at 7.877, 11.871, and 17.876.

In some other embodiments, the X-ray powder diffraction pattern expressed in terms of 2*θ* angles, which are diffraction angles, of the crystal form A has characteristic peaks at 7.877, 9.115, 11.871, and 17.876.

In some other embodiments, the X-ray powder diffraction pattern expressed in terms of 2*θ* angles, which are diffraction angles, of the crystal form A has characteristic peaks at 7.877, 9.115, 11.871, 15.851, 17.876, and 18.900.

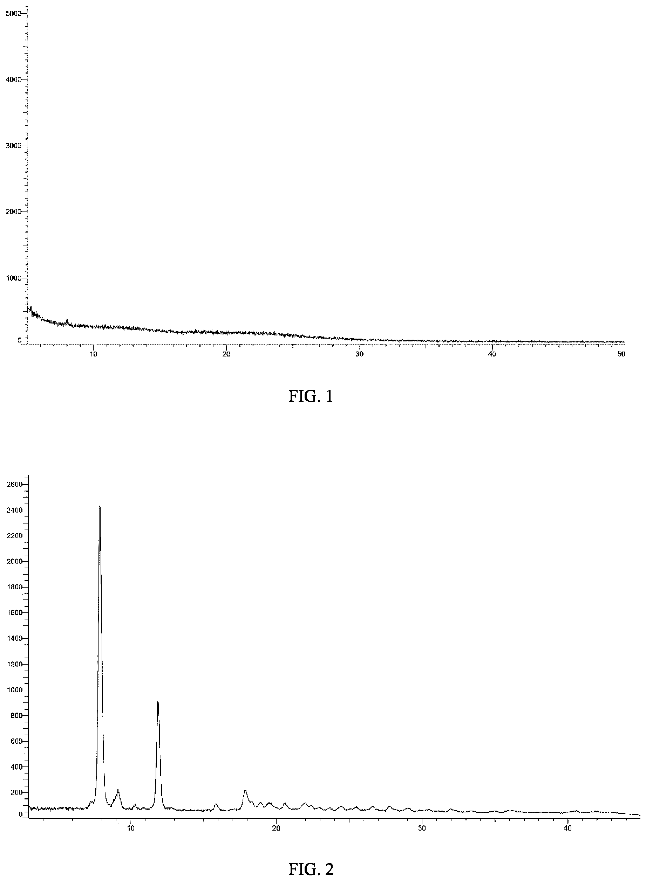

In some other embodiments, the X-ray powder diffraction pattern expressed in terms of 2*θ* angles, which are diffraction angles, of the crystal form A is shown in FIG. 2.

In another aspect, the present disclosure provides a preparation method for the crystal form A of the compound of formula 1, (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, the method comprising: dissolving the compound of formula 1, (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, in a solvent (1), then adding a solvent (2), and stirring for crystallization.

In some embodiments, the solvent (1) is selected from the group consisting of at least one of the solvents dichloromethane, methanol, water and isopropanol, tetrahydrofuran, ethanol, dimethyl sulfoxide, and N,N-dimethylformamide.

In some embodiments, the solvent (2) is selected from the group consisting of acetone, water, acetonitrile, methyl tert-butyl ether, n-heptane, isopropyl acetate.

In another aspect, the present disclosure provides a preparation method for the crystal form A of the compound of formula 1, (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, the method comprising: mixing the compound of formula 1, (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, with a solvent (3) and stirring for crystallization.

In some embodiments, the solvent (3) is selected from the group consisting of water, methanol, ethanol, isopropanol, n-propanol, acetone, ethyl acetate, acetonitrile, isopropyl acetate, methyl tert-butyl ether, 2-butanone, tetrahydrofuran, n-heptane, 1,4-dioxane, isopentanol, methanol/water (1:1), ethyl acetate/ethanol (1:1), ethyl acetate/n-heptane (1:1), cyclohexane, isopropyl ether, propylene glycol methyl ether, 10% water/methanol, 7% water/ethanol, 10% water/isopropanol, 10% water/acetone, acetonitrile/methanol (v/v = 1:1), and tetrahydrofuran/ethanol (v/v = 2:1)).

In another aspect, the present disclosure provides a preparation method for the crystal form A of the compound of formula 1, (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, the method comprising: dissolving the compound of formula 1, (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, in (4) and volatilizing for crystallization.

In some embodiments, the solvent (4) is selected from the group consisting of DMSO, dichloromethane, trichloromethane, N,N-dimethylformamide, N,N-dimethylacetamide, and DMSO/tetrahydrofuran (v/v = 1:5).

In another aspect, the present disclosure provides a crystal form B of the compound of formula 1, (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide. In some embodiments, an X-ray powder diffraction pattern expressed in terms of 2*θ* angles, which are diffraction angles, of the crystal form B has characteristic peaks at 8.002, 9.248, 11.985, 13.412, and 17.526.

In some other embodiments, the X-ray powder diffraction pattern expressed in terms of 2*θ* angles, which are diffraction angles, of the crystal form B has characteristic peaks at 8.002, 9.248, 11.985, 13.412, 17.526, and 17.929.

In some other embodiments, the X-ray powder diffraction pattern expressed in terms of 2*θ* angles, which are diffraction angles, of the crystal form B has characteristic peaks at 8.002, 8.978, 9.248, 11.985, 13.412, 17.526, and 17.929.

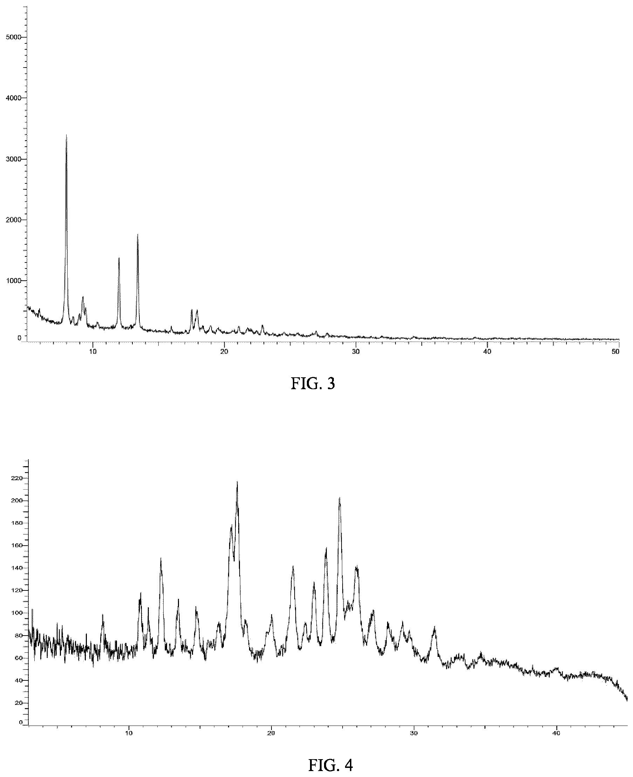

In some other embodiments, the X-ray powder diffraction pattern expressed in terms of 2*θ* angles, which are diffraction angles, of the crystal form B is shown in FIG. 3.

In another aspect, the present disclosure provides a pharmaceutically acceptable salt of the compound of formula 1, (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, wherein the pharmaceutically acceptable salt is selected from the group consisting of hydrochloride, sulfate, phosphate, mesylate, succinate, fumarate, maleate, p-toluenesulfonate, L-tartrate, D-malate, L-malate, and citrate.

In an optional embodiment, a chemical ratio of the compound of formula 1, (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, to acid is 3:1-1:3, including but not limited to 3:1, 2:1, 1:1, 1:2, and 1:3.

In another embodiment, a chemical ratio of the compound of formula 1, (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, to acid is 2:1-1:2.

In an optional embodiment, a chemical ratio of the compound of formula 1, (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, to hydrochloric acid is 1:1 or 1:2.

In an optional embodiment, a chemical ratio of the compound of formula 1, (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, to sulfuric acid is 1:1 or 2:1.

In an optional embodiment, a chemical ratio of the compound of formula 1, (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, to phosphoric acid is 1:1.

In an optional embodiment, a chemical ratio of the compound of formula 1, (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, to methanesulfonic acid is 1:1.

In an optional embodiment, a chemical ratio of the compound of formula 1, (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, to succinic acid is 2:1 or 1:1.

In an optional embodiment, a chemical ratio of the compound of formula 1, (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, to fumaric acid is 2:1 or 1:1.

In an optional embodiment, a chemical ratio of the compound of formula 1, (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, to maleic acid is 2:1 or 1:1.

In an optional embodiment, a chemical ratio of the compound of formula 1, (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, to p-toluenesulfonic acid is 1:1.

In an optional embodiment, a chemical ratio of the compound of formula 1, (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, to L-tartaric acid is 1:1-1:2.

The present disclosure further provides a preparation method for a pharmaceutically acceptable salt of (*R*)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, the method comprising a step of reacting (*R*)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide with an acid, wherein the acid is selected from the group consisting of hydrochloric acid, sulfuric acid, phosphoric acid, methanesulfonic acid, succinic acid, fumaric acid, maleic acid, p-toluenesulfonic acid, L-tartaric acid, D-malic acid, L-malic acid, and citric acid.

Solvents used for salt formation in the present disclosure are selected from the group consisting of, but are not limited to, acetonitrile, acetone, tetrahydrofuran, ethanol, methanol, 1,4-dioxane, dichloromethane/methanol, water/isopropanol, and tetrahydrofuran/ethanol.

Further, in an optional embodiment, the method for preparing the aforementioned pharmaceutically acceptable salt further comprises such a step as crystallization, filtration, washing, or drying.

In another aspect, the present disclosure further provides a crystal form a of hydrochloride of the compound of formula 1, (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, wherein an X-ray powder diffraction pattern expressed in terms of 2*θ* angles, which are diffraction angles, of the crystal form has characteristic peaks at 8.164, 10.795, 11.375, 12.281, 13.481, 14.813, 17.195, and 17.606.

In some other embodiments, the X-ray powder diffraction pattern expressed in terms of 2*θ* angles, which are diffraction angles, of the crystal form a of hydrochloride has characteristic peaks at 8.164, 10.795, 11.375, 12.281, 13.481, 14.813, 17.195, 17.606, 21.523, 22.982, 23.845, 24.814, and 25.962.

In some other embodiments, the X-ray powder diffraction pattern expressed in terms of 2*θ* angles, which are diffraction angles, of the crystal form a of hydrochloride has characteristic peaks at 8.164, 10.795, 11.375, 12.281, 13.481, 14.813, 17.195, 17.606, 18.208, 20.026, 21.523, 22.982, 23.845, 24.814, 25.363, 25.962, 27.149, and 29.207.

In some other embodiments, the X-ray powder diffraction pattern expressed in terms of 2*θ* angles, which are diffraction angles, of the crystal form a of hydrochloride is shown in FIG. 4.

The present disclosure further provides a method for preparing the crystal form a of hydrochloride of the compound of formula 1, (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, the method comprising: dissolving the compound of formula 1, (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, in tetrahydrofuran/ethanol (v/v = 2:1), adding hydrochloric acid, and stirring.

The present disclosure further provides a crystal form b of hydrochloride of the compound of formula 1, (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, wherein an X-ray powder diffraction pattern expressed in terms of 2*θ* angles, which are diffraction angles, of the crystal form has characteristic peaks at 5.166, 7.759, 10.741, 14.887, 15.685, 16.604, 17.641, 21.855, and 26.448.

In some embodiments, the X-ray powder diffraction pattern expressed in terms of 2*θ* angles, which are diffraction angles, of the crystal form b of hydrochloride has characteristic peaks at 5.166, 7.759, 10.741, 14.201, 14.887, 15.685, 16.604, 17.641, 18.963, 21.855, 22.337, 22.949, and 26.448.

In some embodiments, the X-ray powder diffraction pattern expressed in terms of 2*θ* angles, which are diffraction angles, of the crystal form b of hydrochloride has characteristic peaks at 5.166, 7.759, 10.741, 14.201, 14.887, 15.685, 16.604, 17.641, 18.963, 21.855, 22.337, 22.949, 24.269, 26.448, 27.540, 28.187, 28.606, and 29.149.

In some embodiments, the X-ray powder diffraction pattern expressed in terms of 2*θ* angles, which are diffraction angles, of the crystal form b of hydrochloride has characteristic peaks at 5.166, 7.759, 10.741, 14.201, 14.887, 15.685, 16.604, 17.641, 18.963, 21.059, 21.855, 22.337, 22.949, 24.269, 25.838, 26.448, 27.540, 28.187, 28.606, 29.149, 30.188, 32.330, 33.121, 33.450, 33.780, and 34.670.

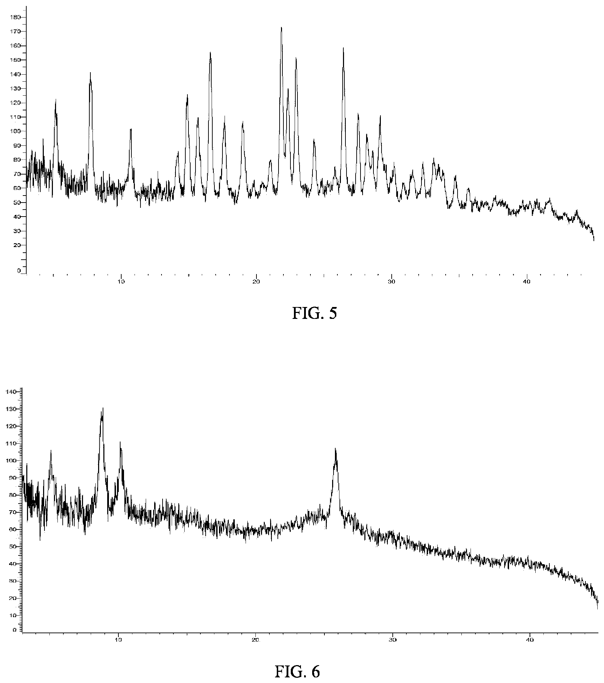

In some other embodiments, the X-ray powder diffraction pattern expressed in terms of 2*θ* angles, which are diffraction angles, of the crystal form b of hydrochloride is shown in FIG. 5.

The present disclosure further provides a method for preparing the crystal form b of hydrochloride of the compound of formula 1, (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, the method comprising: dissolving the compound of formula 1, (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, in dichloromethane/methanol (v/v = 2:1), adding hydrochloric acid, and stirring.

The present disclosure further provides a crystal form c of hydrochloride of the compound of formula 1, (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, wherein an X-ray powder diffraction pattern expressed in terms of 2*θ* angles, which are diffraction angles, of the crystal form has characteristic peaks at 5.095, 8.779, 10.203, and 25.834.

In some other embodiments, the X-ray powder diffraction pattern expressed in terms of 2*θ* angles, which are diffraction angles, of the crystal form c of hydrochloride is shown in FIG. 6.

The present disclosure further provides a method for preparing the crystal form c of hydrochloride of the compound of formula 1, (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, the method comprising: dissolving the compound of formula 1, (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, in 10% water/isopropanol, adding hydrochloric acid, adding isopropyl acetate, and stirring.

The present disclosure further provides a crystal form α of sulfate of the compound of formula 1, (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, wherein an X-ray powder diffraction pattern expressed in terms of 2*θ* angles, which are diffraction angles, of the crystal form has characteristic peaks at 5.180, 8.955, 10.380, 13.767, and 15.578.

In some embodiments, the X-ray powder diffraction pattern expressed in terms of 2*θ* angles, which are diffraction angles, of the crystal form α of sulfate has characteristic peaks at 5.180, 8.955, 10.380, 13.767, 15.578, and 25.809.

In some embodiments, the X-ray powder diffraction pattern expressed in terms of 2*θ* angles, which are diffraction angles, of the crystal form α of sulfate has characteristic peaks at 5.180, 8.955, 10.380, 13.767, 15.578, 18.063, 18.781, and 25.809.

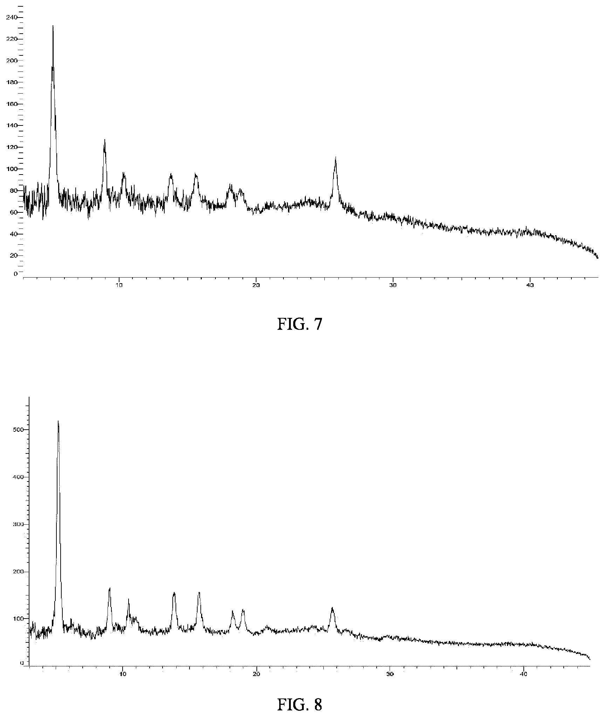

In some other embodiments, the X-ray powder diffraction pattern expressed in terms of 2*θ* angles, which are diffraction angles, of the crystal form α of sulfate is shown in FIG. 7.

The present disclosure further provides a method for preparing the crystal form α of sulfate of the compound of formula 1, (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, the method comprising: dissolving the compound of formula 1, (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, in a solvent (5), adding sulfuric acid, and stirring.

The solvent (5) described in the present disclosure is selected from the group consisting of 10% water/isopropanol, tetrahydrofuran/ethanol (v/v = 2:1), and dichloromethane/methanol (v/v = 2:1).

The present disclosure further provides a crystal form α of phosphate of the compound of formula 1, (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, wherein an X-ray powder diffraction pattern expressed in terms of 2*θ* angles, which are diffraction angles, of the crystal form has characteristic peaks at 5.175, 9.006, 10.437, 13.863, 15.707, and 18.979.

In some embodiments, the X-ray powder diffraction pattern expressed in terms of 2*θ* angles, which are diffraction angles, of the crystal form α of phosphate has characteristic peaks at 5.175, 9.006, 10.437, 10.999, 13.863, 15.707, 18.225, and 18.979.

In some embodiments, the X-ray powder diffraction pattern expressed in terms of 2*θ* angles, which are diffraction angles, of the crystal form α of phosphate has characteristic peaks at 5.175, 9.006, 10.437, 10.999, 13.863, 15.707, 18.225, 18.979, 20.767, and 25.682.

In some other embodiments, the X-ray powder diffraction pattern expressed in terms of 2*θ* angles, which are diffraction angles, of the crystal form α of phosphate is shown in FIG. 8.

The present disclosure further provides a method for preparing the crystal form α of phosphate of the compound of formula 1, (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, the method comprising: dissolving the compound of formula 1, (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, in 10% water/isopropanol or tetrahydrofuran/ethanol (v/v = 2:1), adding phosphoric acid, and stirring.

The present disclosure further provides a crystal form I of succinate of the compound of formula 1, (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, wherein an X-ray powder diffraction pattern expressed in terms of 2*θ* angles, which are diffraction angles, of the crystal form has characteristic peaks at 6.106, 8.589, 12.276, 14.812, 17.517, and 20.400.

In some embodiments, the X-ray powder diffraction pattern expressed in terms of 2*θ* angles, which are diffraction angles, of the crystal form I of succinate has characteristic peaks at 6.106, 8.589, 9.156, 10.134, 12.276, 14.812, 17.517, 20.400, and 24.213.

In some embodiments, the X-ray powder diffraction pattern expressed in terms of 2*θ* angles, which are diffraction angles, of the crystal form I of succinate has characteristic peaks at 6.106, 7.931, 8.589, 9.156, 10.134, 12.276, 14.812, 15.357, 17.517, 20.400, 24.213, 29.362, and 38.514.

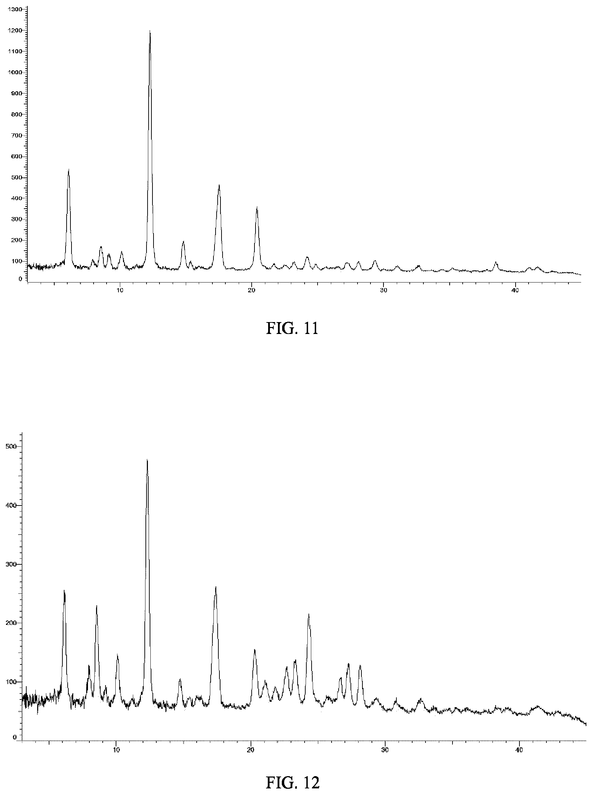

In some embodiments, the X-ray powder diffraction pattern expressed in terms of 2*θ* angles, which are diffraction angles, the X-ray powder diffraction pattern expressed in terms of 2*θ* angles, which are diffraction angles, of the crystal form I of succinate is shown in FIG. 11.

The present disclosure further provides a method for preparing the crystal form I of succinate of the compound of formula 1, (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, the method comprising: dissolving the compound of formula 1, (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, in dichloromethane/methanol (v/v = 2:1), adding succinic acid, and stirring.

The present disclosure further provides a crystal form I of fumarate of the compound of formula 1, (*R*)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, wherein an X-ray powder diffraction pattern expressed in terms of 2*θ* angles, which are diffraction angles, of the crystal form has characteristic peaks at 6.163, 8.552, 12.317, 17.407, and 24.336.

In some embodiments, the X-ray powder diffraction pattern expressed in terms of 2*θ* angles, which are diffraction angles, of the crystal form I of fumarate has characteristic peaks at 6.163, 8.552, 10.102, 12.317, 17.407, 20.302, 23.331, 24.336, 27.279, and 28.148.

In some embodiments, the X-ray powder diffraction pattern expressed in terms of 2*θ* angles, which are diffraction angles, of the crystal form I of fumarate has characteristic peaks at 6.163, 7.982, 8.552, 10.102, 12.317, 14.750, 17.407, 20.302, 21.069, 22.678, 23.331, 24.336, 26.692, 27.279, and 28.148.

In some embodiments, the X-ray powder diffraction pattern expressed in terms of 2*θ* angles, which are diffraction angles, the X-ray powder diffraction pattern expressed in terms of 2*θ* angles, which are diffraction angles, of the crystal form I of fumarate is shown in FIG. 12.

The present disclosure further provides a method for preparing the crystal form I of fumarate of the compound of formula 1, (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, the method comprising: dissolving the compound of formula 1, (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, in a solvent (6), adding fumaric acid, and stirring. The solvent (6) is selected from the group consisting of tetrahydrofuran/ethanol (v/v = 2:1), 10% water/isopropanol, and dichloromethane/methanol (v/v = 2:1).

The present disclosure further provides a crystal form i of hemifumarate of the compound of formula 1, (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, wherein an X-ray powder diffraction pattern expressed in terms of 2*θ* angles, which are diffraction angles, of the crystal form has characteristic peaks at 3.95, 7.89, 8.43, 10.97, 11.85, and 14.17.

In some embodiments, the X-ray powder diffraction pattern expressed in terms of 2*θ* angles, which are diffraction angles, of the crystal form i of hemifumarate has characteristic peaks at 3.95, 6.83, 7.30, 7.89, 8.43, 10.58, 10.97, 11.85, and 14.17.

In some embodiments, the X-ray powder diffraction pattern expressed in terms of 2*θ* angles, which are diffraction angles, of the crystal form i of hemifumarate has characteristic peaks at 3.95, 6.83, 7.30, 7.89, 8.43, 9.95, 10.58, 10.97, 11.85, and 14.17.

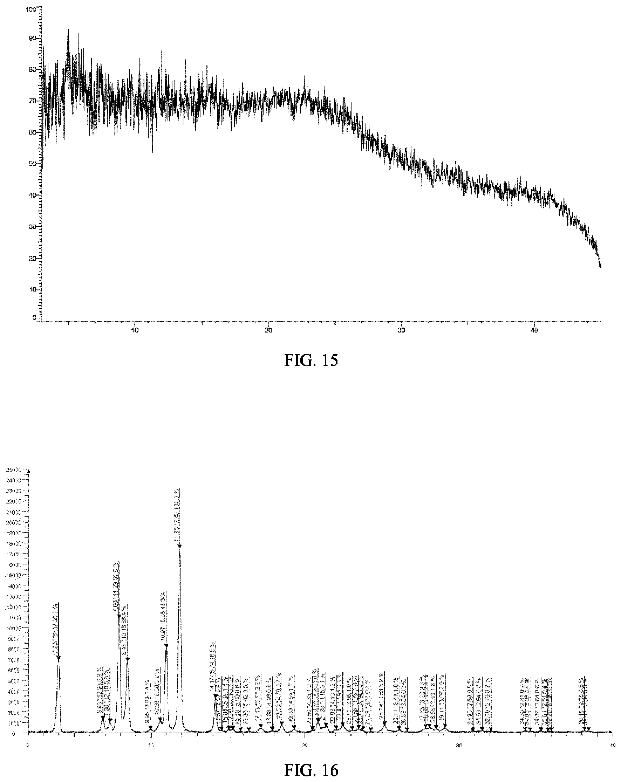

In some embodiments, the X-ray powder diffraction pattern expressed in terms of 2*θ* angles, which are diffraction angles, the X-ray powder diffraction pattern expressed in terms of 2*θ* angles, which are diffraction angles, of the crystal form i of hemifumarate is shown in FIG. 16.

The present disclosure further provides a crystal form ii of hemifumarate of the compound of formula 1, (*R*)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, wherein an X-ray powder diffraction pattern expressed in terms of 2*θ* angles, which are diffraction angles, of the crystal form has characteristic peaks at 8.581, 9.892, 17.287, and 20.078.

In some embodiments, the X-ray powder diffraction pattern expressed in terms of 2*θ* angles, which are diffraction angles, of the crystal form ii of hemifumarate has characteristic peaks at 8.581, 9.892, 12.605, 14.336, 15.293, 17.287, 18.293, 19.342, 20.078, and 22.996.

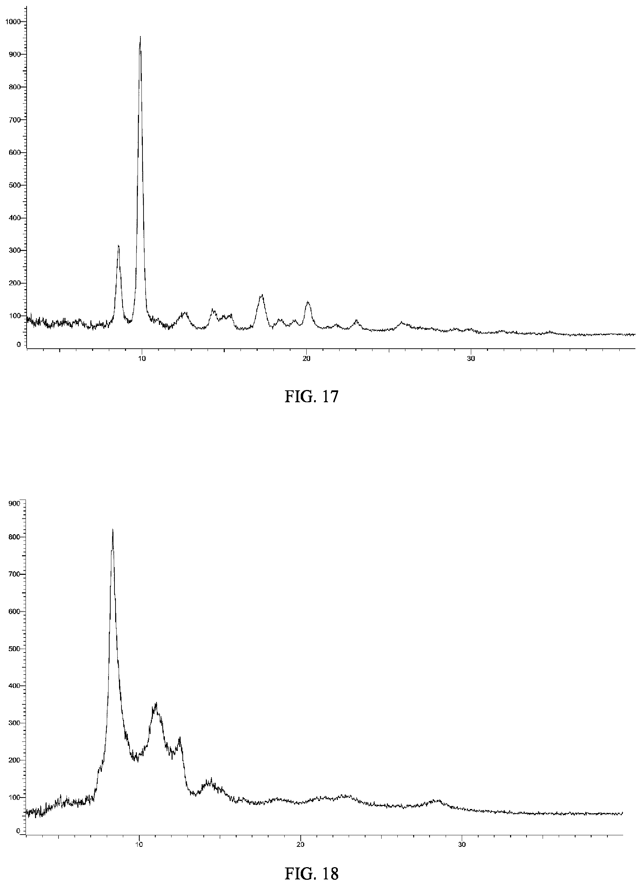

In some embodiments, the X-ray powder diffraction pattern expressed in terms of 2*θ* angles, which are diffraction angles, the X-ray powder diffraction pattern expressed in terms of 2*θ* angles, which are diffraction angles, of the crystal form ii of hemifumarate is shown in FIG. 17.

The present disclosure further provides a crystal form iii of hemifumarate of the compound of formula 1, (*R*)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, wherein an X-ray powder diffraction pattern expressed in terms of 2*θ* angles, which are diffraction angles, of the crystal form has characteristic peaks at 8.363, 11.005, 12.498, 14.267, and 28.341.

In some embodiments, the X-ray powder diffraction pattern expressed in terms of 2*θ* angles, which are diffraction angles, the X-ray powder diffraction pattern expressed in terms of 2*θ* angles, which are diffraction angles, of the crystal form iii of hemifumarate is shown in FIG. 18.

The present disclosure further provides a crystal form a of maleate of the compound of formula 1, (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, wherein an X-ray powder diffraction pattern expressed in terms of 2*θ* angles, which are diffraction angles, of the crystal form has characteristic peaks at 7.902, 9.389, 11.879, 15.683, and 21.632.

In some embodiments, the X-ray powder diffraction pattern expressed in terms of 2*θ* angles, which are diffraction angles, of the crystal form a of maleate has characteristic peaks at 7.902, 8.629, 9.109, 9.389, 11.879, 15.683, 17.043, 17.871, 20.015, 21.632, and 25.672.

In some embodiments, the X-ray powder diffraction pattern expressed in terms of 2*θ* angles, which are diffraction angles, of the crystal form a of maleate has characteristic peaks at 7.902, 8.629, 9.109, 9.389, 11.879, 13.509, 14.285, 15.683, 17.043, 17.453, 17.871, 19.572, 20.015, 21.632, 24.489, and 25.672.

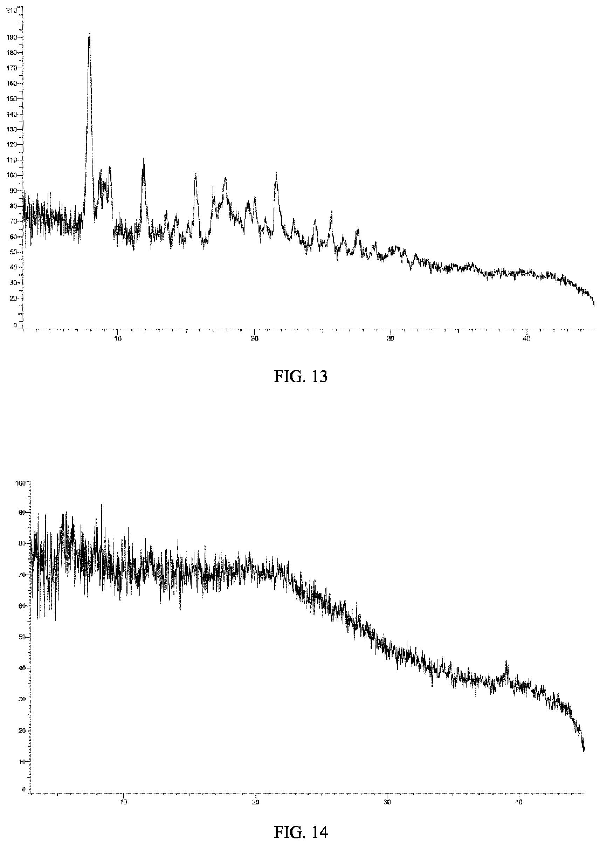

In some embodiments, the X-ray powder diffraction pattern expressed in terms of 2*θ* angles, which are diffraction angles, the X-ray powder diffraction pattern expressed in terms of 2*θ* angles, which are diffraction angles, of the crystal form a of maleate is shown in FIG. 13.

The present disclosure further provides a method for preparing the crystal form a of maleate of the compound of formula 1, (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, the method comprising: dissolving the compound of formula 1, (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, in dichloromethane/methanol (v/v = 2:1), adding maleic acid, then adding methyl tert-butyl ether, and stirring for crystallization.

Further, provided are the crystal forms of the compound of formula 1, (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, of the present disclosure, wherein the 2*θ* angles have a margin of error of ±0.2.

In certain embodiments, the preparation methods for the crystal forms described in the present disclosure further comprise a crystallization, filtration, washing, or drying step. In another aspect, the present disclosure further provides a pharmaceutical composition comprising the aforementioned crystal form A of the compound of formula 1, (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, or a pharmaceutically acceptable salt of the compound of formula 1, (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, or a crystal form thereof, and optionally a pharmaceutically acceptable excipient.

The present disclosure further provides a preparation method for a pharmaceutical composition, the method comprising a step of mixing the aforementioned crystal form A of the compound of formula 1, (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, or a pharmaceutically acceptable salt of the compound of formula 1, (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, or a crystal form thereof, with a pharmaceutically acceptable excipient. The present disclosure further provides use of the aforementioned crystal form A or crystal form B of the compound of formula 1, (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, or a pharmaceutically acceptable salt of the compound of formula 1, (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, or a crystal form thereof, or the aforementioned pharmaceutical composition, as a PARP1 inhibitor.

The present disclosure further provides use of the aforementioned crystal form A or crystal form B of the compound of formula 1, (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, or a pharmaceutically acceptable salt of the compound of formula 1, (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, or a crystal form thereof, or the aforementioned pharmaceutical composition, in the preparation of a PARP1 inhibitor medicament.

The present disclosure further provides use of the aforementioned crystal form A or crystal form B of the compound of formula 1, (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, or a pharmaceutically acceptable salt of the compound of formula 1, (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, or a crystal form thereof, or the aforementioned pharmaceutical composition, for treating and/or preventing a cancer.

The present disclosure further provides use of the aforementioned crystal form A or crystal form B of the compound of formula 1, (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, or a pharmaceutically acceptable salt of the compound of formula 1, (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, or a crystal form thereof, or the aforementioned pharmaceutical composition, in the preparation of a medicament for treating and/or preventing a cancer.

Provided are the uses described in the present disclosure, wherein the cancer is selected from the group consisting of breast cancer, ovarian cancer, pancreatic cancer, prostate cancer, gastric cancer, colorectal cancer, lung cancer, renal cancer, hepatic cancer, cervical cancer, endometrial cancer, myeloma, leukemia, lymphoma, acoustic neuroma, basal cell carcinoma, cholangiocarcinoma, bladder cancer, brain cancer, bronchogenic carcinoma, sarcoma, chordoma, choriocarcinoma, craniopharyngioma, cystadenocarcinoma, embryonal carcinoma, hemangioendothelioma, ependymoma, epithelial cancer, esophageal cancer, essential thrombocytosis, Ewing sarcoma, testicular cancer, glioma, heavy chain disease, hemangioblastoma, medullary carcinoma, medulloblastoma, melanoma, meningioma, mesothelioma, neuroblastoma, NUT midline carcinoma, neuroglioma, bone cancer, nasopharyngeal carcinoma, oral cancer, thyroid cancer, pinealoma, polycythemia vera, retinoblastoma, sebaceous carcinoma, seminoma, skin cancer, squamous cell carcinoma, synovioma, sweat gland carcinoma, Waldenström macroglobulinemia, and Wilms tumor; preferably, the cancer is selected from the group consisting of breast cancer, ovarian cancer, pancreatic cancer, prostate cancer, gastric cancer, colorectal cancer, and lung cancer.

As used herein, "2*θ* or 2*θ* angle" refers to a diffraction angle; *θ* refers to the Bragg angle in ° or degrees; the 2*θ* of each characteristic peak has a margin of error of ±0.20 (including the case that a number having more than 1 decimal place is rounded), specifically -0.20, -0.19, -0.18, -0.17, -0.16, -0.15, -0.14, -0.13, -0.12, -0.11, -0.10, -0.09, -0.08, -0.07, -0.06, -0.05, -0.04, -0.03, -0.02, -0.01, 0.00, 0.01, 0.02, 0.03, 0.04, 0.05, 0.06, 0.07, 0.08, 0.09, 0.10, 0.11, 0.12, 0.13, 0.14, 0.15, 0.16, 0.17, 0.18, 0.19, or 0.20.

In the present disclosure, there is a certain degree of error in the measurement of the chemical ratio of the compound to the acid molecule. Generally, ±10% is considered a reasonable margin of error. The error varies to some extent depending on the context in which it is used, and the variation does not exceed ±10% and may be ±9%, ±8%, ±7%, ±6%, ±5%, ±4%, ±3%, ±2%, or ±1%, preferably ±5%. The numerical values described with "about" in the present disclosure have the aforementioned reasonable margin of error.

As used herein, "crystallization" or "crystallizing" includes, but is not limited to, stirring crystallization, slurrying crystallization, cooling crystallization, and volatilizing crystallization.

As used herein, "differential scanning calorimetry" or "DSC" refers to the measurement of the temperature difference and heat flow difference between a sample and a reference substance during a ramping or thermostatic process to characterize all the physical changes and chemical changes related to the thermal effect, and to obtain phase change information about the sample.

In the present disclosure, the drying temperature is generally 25 °C-100 °C, preferably 40 °C-70 °C, and the drying may be performed under atmospheric pressure or reduced pressure.

As used herein, "pharmaceutically acceptable excipient" includes, but is not limited to, any auxiliary, carrier, glidant, sweetener, diluent, preservative, dye/colorant, flavoring agent, surfactant, wetting agent, dispersant, suspending agent, stabilizer, isotonic agent, or emulsifier that has been approved by the U.S. Food and Drug Administration as acceptable for use in humans or livestock animals.

### BRIEF DESCRIPTION OF THE DRAWINGS

FIG. 1 shows an XRPD pattern of the amorphous form of compound 1.
FIG. 2 shows an XRPD pattern of the crystal form A of compound 1.
FIG. 3 shows an XRPD pattern of the crystal form B of compound 1.
FIG. 4 shows an XRPD pattern of the crystal form a of hydrochloride of compound 1.
FIG. 5 shows an XRPD pattern of the crystal form b of hydrochloride of compound 1.
FIG. 6 shows an XRPD pattern of the crystal form c of hydrochloride of compound 1.
FIG. 7 shows an XRPD pattern of the crystal form α of sulfate of compound 1.
FIG. 8 shows an XRPD pattern of the crystal form α of phosphate of compound 1.
FIG. 9 shows an XRPD pattern of the amorphous phosphate of compound 1.
FIG. 10 shows an XRPD pattern of the amorphous mesylate of compound 1.
FIG. 11 shows an XRPD pattern of the crystal form I of succinate of compound 1.
FIG. 12 shows an XRPD pattern of the crystal form I of fumarate of compound 1.
FIG. 13 shows an XRPD pattern of the crystal form a of maleate of compound 1.
FIG. 14 shows an XRPD pattern of the amorphous p-toluenesulfonate of compound 1.
FIG. 15 shows an XRPD pattern of the amorphous L-tartrate of compound 1.
FIG. 16 shows an XRPD pattern of the crystal form i of hemifumarate of compound 1.
FIG. 17 shows an XRPD pattern of the crystal form ii of hemifumarate of compound 1.
FIG. 18 shows an XRPD pattern of the crystal form iii of hemifumarate of compound 1.

### DETAILED DESCRIPTION

The present disclosure is further illustrated in detail by the following examples and experimental examples. These examples and experimental examples are illustrative only and are not intended to limit the scope of the present disclosure.

Test conditions for the instruments used in the experiments:
The structures of the compounds were determined by nuclear magnetic resonance (NMR) spectroscopy or/and mass spectrometry (MS). The NMR shifts (δ) are given in 10⁻⁶ (ppm). The NMR analyses were performed using a Bruker AVANCE-400 nuclear magnetic resonance instrument, with dimethyl sulfoxide-D6 (DMSO-*d*₆), chloroform-D (CDCl₃), and methanol-D4 (CD₃OD) as solvents and tetramethylsilane (TMS) as an internal standard.

The MS analyses were performed using an Agilent 1200/1290 DAD-6110/6120 Quadrupole MS liquid chromatography-mass spectrometry system (manufacturer: Agilent; MS model: 6110/6120 Quadrupole MS), waters ACQuity UPLC-QD/SQD (manufacturer: waters; MS model: waters ACQuity Qda Detector/waters SQ Detector), and THERMO Ultimate 3000-Q Exactive (manufacturer: THERMO; MS model: THERMO Q 15 Exactive).

The HPLC analyses were performed using an Agilent 1260DAD high pressure liquid chromatograph (Sunfire C18 150 × 4.6 mm chromatography column) and a Thermo U3000 high pressure liquid chromatograph (Gimini C18 150 × 4.6 mm chromatography column).

XRPD refers to X-ray powder diffraction: The analysis was performed using a BRUKER D8 X-ray diffractometer; specific acquisition information: a Cu anode (40 kV, 40 mA), ray: monochromatic Cu-Ka ray (1 = 1.5418 Å). Scan mode: *θ*/2*θ*; scan range: 3-48°. XRPD refers to X-ray powder diffraction: The analysis was performed using a BRUKER D8 FOCUS X-ray diffractometer; specific acquisition information: ray: monochromatic Cu-Ka ray (1 = 1.5418 Å). Scan mode: *θ*/2*θ*; scan range: 2-40°.

DSC refers to differential scanning calorimetry: The measurement was performed using a METTLER TOLEDO DSC 3+ differential scanning calorimeter, with a temperature ramp rate of 10 °C/min, 25-350 °C, and a nitrogen purge speed of 50 mL/min.

TGA refers to thermogravimetric analysis: The analysis was performed using a METTLER TOLEDO TGA 2 thermogravimetric analyzer, with a temperature ramp rate of 10 °C/min, specific temperature ranges shown in corresponding patterns, and a nitrogen purge speed of 50 mL/min.

DVS refers to dynamic vapor sorption: Surface Measurement Systems instrinsic was adopted; the humidity started at 50%, the humidity range of the investigation was 0%-95%, and the humidity was increased in increments of 10%; the criterion was that each gradient mass change dM/dT is ≤ 0.002%, TMAX 360 min, two cycles.

The known starting materials in the present disclosure may be synthesized by using or according to methods known in the art, or may be purchased from companies such as ABCR GmbH & Co. KG, Acros Organics, Aldrich Chemical Company, Accela ChemBio Inc., and Chembee Chemicals.

The monitoring of the reaction progress in the examples was performed by thin-layer chromatography (TLC). The developing solvents for the reactions, the eluent systems for the column chromatography purification, and the developing solvent systems for the thin-layer chromatography include: A: a dichloromethane/methanol system, and B: a n-hexane/ethyl acetate system. The volume ratio of the solvents was adjusted depending on the polarity of the compound, or small amounts of basic or acidic reagents such as triethylamine and acetic acid may also be added for adjustments.

### Example 1. Preparation of Compound 1 (with reference to the preparation method of Example 8 in Application No. PCT/CN2022/094612)

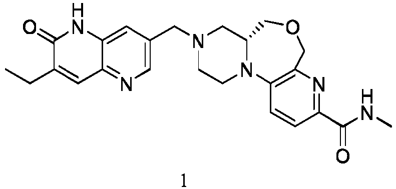

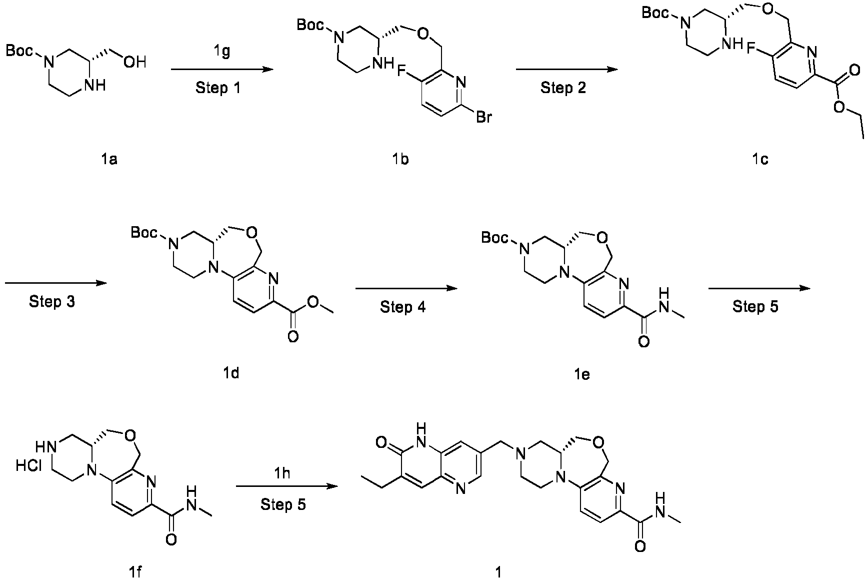

### Step 1

### tert-Butyl (R)-3-(((6-bromo-3-fluoropyridin-2-yl)methoxy)methyl)piperazine-1-carboxylate 1b

The compound 6-bromo-2-(bromomethyl)-3-fluoropyridine **1g** (2.5 g, 9.29 mmol, prepared using the method disclosed in Preparation Example 6 on page 12 of the specification in the patent application "WO2016077161A1") and the compound tert-butyl (*R*)-3-(hydroxymethyl)piperazine-1-carboxylate **1a** (2.25 g, 10.40 mmol, Shanghai Hanhong) were dissolved in tetrahydrofuran (30 mL), and sodium hydride (812.5 mg, 21.20 mmol, 60% purity) was added under an ice bath. The mixture was stirred for 2 h. The reaction mixture was concentrated under reduced pressure, and the residue was purified by silica gel column chromatography using eluent system A to give the title compound **1b** (3 g, yield: 79.8%).

MS m/z (ESI): 404.1 [M+1].

### Step 2

### tert-Butyl (R)-3-(((6-(ethoxycarbonyl)-3-fluoropyridin-2-yl)methoxy)methyl)piperazine-1-carboxylate 1c

Compound **1b** (2 g, 4.94 mmol) was dissolved in a mixed solvent of *N,N-*dimethylformamide (20 mL) and ethanol (10 mL), and bis(triphenylphosphine)palladium(II) dichloride (0.52 g, 740.8 µmol) and *N,N-*diisopropylethylamine (1.52 g, 15 mmol) were added. The mixture was stirred at 100 °C for 14 h under a carbon monoxide atmosphere. The reaction mixture was cooled, then diluted with ethyl acetate (100 mL), and washed with water and a saturated sodium chloride solution in sequence. The organic phase was collected, dried over anhydrous sodium sulfate, and filtered to remove the desiccant. Then the filtrate was concentrated under reduced pressure, and the residue was purified by silica gel column chromatography using eluent system B to give the title compound **1c** (1.5 g, yield: 76.2%).

MS m/z (ESI): 398.2 [M+1].

### Step 3

### 3-(tert-Butyl) 9-methyl-(R)-1,2,4a,5-tetrahydro-7H-pyrazino[2,1-c]pyrido[3,2-e][1,4]oxazepine-3,9(4H)-dicarboxylate 1d

Compound **1c** (4 g, 10.06 mmol) was dissolved in *N,N-*dimethylacetamide (20 mL), and *N,N*-diisopropylethylamine (4 g, 30.9 mmol) was added. The mixture was microwaved at 140 °C for 6 h. The reaction mixture was cooled and then concentrated under reduced pressure, and the residue was purified by silica gel column chromatography using eluent system A to give the title compound **1d** (2.3 g, yield: 60%).

MS m/z (ESI): 364.2 [M+1].

### Step 4

### tert-Butyl (R)-9-(methylaminocarbonyl)-1,2,4a,5-tetrahydro-7H-pyrazino[2,1-c]pyrido[3,2-e][1,4]oxazepine-3(4H)-carboxylate 1e

Compound **1d** (600 mg, 1.58 mmol) was dissolved in a solution of methylamine in ethanol (5 mL, 1 M), and the mixture was stirred for 14 h. The reaction mixture was concentrated under reduced pressure to give crude title compound **1e** (570 mg, yield: 98%). The product was directly used in the next step without purification.

MS m/z (ESI): 363.2 [M+1].

### Step 5

### (R)-N-Methyl-1,2,3,4,4a,5-hexahydro-7H-pyrazino[2,1-c]pyrido[3,2-e][1,4]oxazepine-9-carboxamide hydrochloride 1f

The crude compound **1e** (140 mg, 386.2 µmol) was dissolved in dichloromethane (3 mL), and a 4 M solution of hydrochloric acid in dioxane (1 mL) was added. The mixture was stirred for 2 h. The reaction mixture was concentrated under reduced pressure to give crude title compound **1f** (110 mg, yield: 95%). The product was directly used in the next step without purification.

MS m/z (ESI): 263.2 [M+1].

### Step 6

### (R)-3-((7-Ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide 1

The crude compound **1f** (570 mg, 1.9 mol), the compound 7-(chloromethyl)-3-ethyl-1,5-naphthyridin-2(1H)-one **1h** (430 mg, 1.93 mol, prepared using the method disclosed in Example 4 on page 15 of the specification in the patent application "WO2021013735A1"), and *N,N*-diisopropylethylamine (1.5 g, 11.6 mmol) were dissolved in acetonitrile (30 mL), and sodium iodide (30 mg, 200 µmol) was added. The mixture was left to react at 80 °C for 5 h. After the reaction mixture was concentrated under reduced pressure, the crude product was purified by high performance liquid chromatography (Waters-2545, column: SharpSil-T C18, 30 × 150 mm, 5 µm; mobile phase: aqueous phase (10 mmol/L ammonium bicarbonate) and acetonitrile; gradient ratio: acetonitrile 30%-45%; flow rate: 30 mL/min) to give the title compound 1 (5.4 mg, yield: 8%).

MS m/z (ESI): 449.2 [M+1].

¹H NMR (500 MHz, CD₃OD): δ 8.51 (d, 1H), 7.93 (d, 1H), 7.86 (s, 1H), 7.79 (d, 1H), 7.50 (d, 1H), 4.99 (t, 2H), 4.85 (d, 1H), 4.08 (dd, 2H), 3.87 (dd, 1H), 3.81-3.71 (m, 2H), 3.46 (ddd, 2H), 2.95 (s, 3H), 2.86-2.78 (m, 1H), 2.75-2.61 (m, 3H), 2.57 (dd, 1H), 1.31 (t, 3H).

The X-ray powder diffraction pattern of the amorphous form is shown in FIG. 1.

### Example 2. Cell Proliferation Assay

In the method below, the inhibitory effect of the compound 1 of the present disclosure on the proliferation of DLD1, DLD1^{BRCA2-/-}, and MDA-MB-436 cells was evaluated based on IC₅₀ by determining the ATP content in the cells. Below is a brief description of the experimental method:

### I. Experimental materials and instruments

1. DLD1, human colon cancer tumor cells (Nanjing Cobioer, CBP60037), DLD1^{BRCA2-/-}, human BRCA2 gene knock-out colon cancer cells (Creative biogene, CSC-RT0015)
2. MDA-MB-436, human breast cancer cells (ATCC, HTB-130)
3. Fetal bovine serum (GIBCO, 10091-148)
4. CellTite-Glo reagent (Promega, G7573)
5. 96-well cell culture plates (corning, 3903)
6. Trypsin (invitrogen, 25200-072)
7. Microplate reader (BMG, PHERAsta)
8. Cell counter (Countstar, Shanghai, IC1000)

### II. Experimental procedure

DLD1 cells were cultured in an RPMI-1640 culture medium containing 10% FBS and passaged 2-3 times a week in a passage ratio of 1:6 or 1:8. During the passages, cells were trypsinized, then transferred to a centrifuge tube, and centrifuged at 1200 rpm for 3 min. The supernatant was discarded, and a fresh culture medium was added to resuspend the cells. To a 96-well cell culture plate, 180 µL of the cell suspension was added at a density of 2.78 × 10³ cells/mL. To peripheral wells of the 96-well plate, only 180 µL of a complete culture medium was added.

DLD1^{BRCA2-/-} cells were cultured in an RPMI-1640 culture medium containing 10% FBS and passaged 2-3 times a week in a passage ratio of 1:6 or 1:8. During the passages, cells were trypsinized, then transferred to a centrifuge tube, and centrifuged at 1200 rpm for 3 min. The supernatant was discarded, and a fresh culture medium was added to resuspend the cells. To a 96-well cell culture plate, 180 µL of the cell suspension was added at a density of 8.34 × 10³ cells/mL. To peripheral wells of the 96-well plate, only 180 µL of a complete culture medium was added.

MDA-MB-436 cells were cultured in a Leibovitz's L-15 culture medium containing 10% FBS, 10 µg/mL insulin, and 16 µg/mL glutathione and passaged 2-3 times a week in a passage ratio of 1:3 or 1:5. During the passages, cells were trypsinized, then transferred to a centrifuge tube, and centrifuged at 1200 rpm for 3 min. The supernatant was discarded, and a fresh culture medium was added to resuspend the cells. To a 96-well cell culture plate, 180 µL of the cell suspension was added at a density of 8.34 × 10³ cells/mL. To peripheral wells of the 96-well plate, only 180 µL of a complete culture medium was added.

The culture plates were incubated in an incubator for 24 h (37 °C, 5% CO₂).

The test sample was diluted to 2 mM in DMSO and serially diluted 3-fold to obtain 10 concentrations, and blank and control wells were set. 5 µL of each of the prepared test compound solutions with gradient concentrations was added to 95 µL of a fresh culture medium. Then 20 µL of the above culture medium solution containing the compound was added to the plates. The plates were incubated in an incubator (37 °C, 5% CO₂) for 6 days. 90 µL of CellTiter-Glo reagent was added to each well of the 96-well cell culture plates, and the plates were left to stand at room temperature for 5-10 min in a dark place. The chemiluminescence signal values were read by PHERAstar, and data were processed by GraphPad software. The results are shown in Table 1.

**Table 1. The inhibitory effect of the compound of the present disclosure on the proliferation of DLD1, DLD1^{BRCA2-/-}, and MDA-MB-436 cells**

| Example No. | DLD1, IC₅₀ (nM) | DLD1^{BRCA2-/-} IC₅₀ (nM) | MDA-MB-436 IC₅₀ (nM) |
|---|---|---|---|
| 1 | >1000 | 7.84 | 1.2 |

Conclusion: The compound 1 of the present disclosure has a relatively good inhibitory effect on the proliferation of DLD1^{BRCA2-/-} and MDA-MB-436 cells.

### Example 3. Assay for Binding Activity of Disclosed Compound 1 to PARP1 and PARP2

The *in vitro* binding activity to PARP1 and PARP2 was assessed using the following method.

### I. Experimental materials and instruments

1. PARP1 recombinant protein (Sino Biological, Cat. # 11040-H08B);
2. PARP2 recombinant protein (BPS, Cat. # 80502)
3. Fluorescent probe (made in-house using the compound with the CAS number 1380359-84-1, Shanghai Hengrui); 384-well plates (Corning, 3575)
4. PHERAstar FS microplate reader (BMG Labtech)

### II. Experimental procedure

To each well of a 384-well plate, 8 µL of a binding buffer was added. The fluorescent probe was dissolved in dimethyl sulfoxide, and the solution was diluted to the corresponding concentration. Then the fluorescent probe solution prepared in dimethyl sulfoxide was 20-fold diluted in the binding buffer (50 mM Tris-HCl pH 8.0, 50 mM NaCl, 1 mM MgCl₂, 0.1 mM EDTA, and 0.01% IGEPAL), and the dilution was added at 2 µL/well. The test compound was dissolved in dimethyl sulfoxide, and the solution was diluted to gradient concentrations as required for the experiment. Then the compound solutions with various concentrations prepared in dimethyl sulfoxide were 20-fold diluted in the binding buffer, and the dilutions were added at 2 µL/well. The PARP1 or PARP2 protein was diluted with the binding buffer to the corresponding concentration, and the dilution was added to a black 384-well plate at 8 µL/well. After thorough mixing, the plate was incubated at 25 °C for 40 min. The signal values were read with the FP program in the PHERAstar FS microplate reader. Data were processed using GraphPad software. The binding inhibiting activity of the compound 1 of the present disclosure against PARP1 and PARP2 was assessed through the above assay, and the obtained IC₅₀ values are shown in Table 2.

**Table 2. The binding inhibiting activity of the compound of the present disclosure against PARP1 and PARP2**

| Example No. | PARP1, IC₅₀ (nM) | PARP2, IC₅₀ (nM) |
|---|---|---|
| 1 | 19 | >10000 |

Conclusion: The compound of the present disclosure has a selective inhibitory effect on PARP1.

### Example 4. Preparation of Crystal Form A of Compound of Formula 1

120 mg of the compound represented by formula 1 was dissolved in 2 mL of dichloromethane/methanol (v/v = 1:1), and 2.1 mL of acetone was added. The mixture was stirred for crystallization and filtered, and the solid was dried *in vacuo* to give an off-white solid.

According to X-ray powder diffraction analysis, the product was defined as crystal form A. Its XRPD pattern is shown in FIG. 2, and its characteristic peak positions are shown in Table 3. Its DSC thermogram showed an endothermic peak at 259.82 °C. Its TGA thermogram showed a weight loss of 0.53% from 30 °C to 185 °C.

DVS testing showed that the sample had a hygroscopic weight gain of about 0.31% under normal storage conditions (i.e., 25 °C and 60% RH), a hygroscopic weight gain of about 0.40% under accelerated experimental conditions (i.e., 70% RH), and a hygroscopic weight gain of about 0.76% under extreme conditions (90% RH). In the process of the humidity changing from 0% to 95% RH, the desorption process of the sample substantially overlapped with its adsorption process. In addition, after DVS testing, the crystal form was re-identified, and the results showed that the crystal form did not change.

**Table 3**

| Peak No. | 2θ [° or degrees] | d [Å] | Relative intensity% |
|---|---|---|---|
| 1 | 7.340 | 12.03363 | 1.9 |
| 2 | 7.877 | 11.21434 | 100.0 |
| 3 | 8.857 | 9.97574 | 2.6 |
| 4 | 9.115 | 9.69390 | 5.8 |
| 5 | 10.306 | 8.57657 | 1.7 |
| 6 | 11.871 | 7.44893 | 35.3 |
| 7 | 15.851 | 5.58666 | 2.1 |
| 8 | 17.876 | 4.95789 | 5.9 |
| 9 | 18.252 | 4.85677 | 2.2 |
| 10 | 18.900 | 4.69171 | 1.8 |
| 11 | 19.481 | 4.55302 | 1.8 |
| 12 | 20.591 | 4.30993 | 1.9 |
| 13 | 21.972 | 4.04201 | 1.8 |
| 14 | 22.419 | 3.96246 | 0.9 |
| 15 | 22.935 | 3.87445 | 0.5 |
| 16 | 23.710 | 3.74966 | 0.6 |
| 17 | 24.451 | 3.63768 | 1.1 |
| 18 | 25.463 | 3.49525 | 0.9 |
| 19 | 26.600 | 3.34842 | 1.0 |
| 20 | 27.788 | 3.20791 | 1.4 |
| 21 | 29.157 | 3.06028 | 0.8 |

### Example 5. Preparation of Crystal Form A of Compound of Formula 1

8 mg of the compound represented by formula 1 was added to 0.4 mL of 10% water/isopropanol and dissolved by stirring at 60 °C. The solution was cooled to room temperature, and 1.2 mL of water was added. The mixture was stirred for precipitation and centrifuged, and the solid was dried *in vacuo* and identified as the crystal form A by X-ray powder diffraction.

The solids obtained using the solvents in Table 4 with reference to the above preparation method were identified as the crystal form A by X-ray powder diffraction.

**Table 4. The preparation of the crystal form A of the compound**

| | Compound of formula 1 | Solvent | Procedure |
|---|---|---|---|
| 1 | 8 mg | 0.6 mL of tetrahydrofuran/ethanol (v/v = 2:1) | 1 mL MTBE |
| 2 | | | 1 mL of n-heptane |
| 3 | 30 mg | 2.7 mL of 10% water/methanol | 6 mL of water |
| 4 | | 2.4 mL of 7% water/ethanol | 6 mL of water |
| 5 | | 1.5 mL of 10% water/isopropanol | 6 mL of water |
| 6 | 8 mg | 0.4 mL of 10% water/isopropanol | 1.2 mL of acetonitrile |
| 7 | | | 1.2 mL of acetone |

### Example 6. Preparation of Crystal Form A of Compound of Formula 1

8 mg of the compound represented by formula 1 was dissolved in 0.1 mL of dichloromethane/methanol (v/v = 2:1), and 1 mL of acetone was added. The mixture was stirred for crystallization and centrifuged, and the solid was dried *in vacuo* and identified as the crystal form A by X-ray powder diffraction.

The solids obtained using the solvents in Table 5 with reference to the above preparation method were identified as the crystal form A by X-ray powder diffraction.

**Table 5. The preparation of the crystal form A of the compound**

| NO. | Compound of formula 1 | Solvent | Procedure |
|---|---|---|---|
| 1 | 8 mg | 0.2 mL DMSO | 1 mL of water |
| 2 | | | 1 mL of acetonitrile |
| 3 | | | 1 mL of isopropyl acetate |
| 4 | | | 1 mL of acetone |
| 5 | | 0.1 mL of dichloromethane/methanol (v/v = 2:1) | 1 mL of isopropyl acetate |
| 6 | | | 1 mL of methyl tert-butyl ether |
| 7 | | | 1 mL of n-heptane |
| 8 | 8 mg | 0.8 mL of water | / |
| 9 | | Methanol | |
| 10 | | Ethanol | |
| 11 | | Isopropanol | |
| 12 | | n-Propanol | |
| 13 | | Acetone | |
| 14 | | Ethyl acetate | |
| 15 | | Acetonitrile | |
| 16 | | Isopropyl acetate | |
| 17 | | Methyl tert-butyl ether | |
| 18 | | 2-Butanone | |
| 19 | | Tetrahydrofuran | |
| 20 | | n-Heptane | |
| 21 | | 1,4-Dioxane | |
| 22 | | Isopentanol | |
| 23 | 8 mg | Methanol/water (1:1) | / |
| 24 | | Ethyl acetate/ethanol (1:1) | |
| 25 | | Ethyl acetate/n-heptane (1:1) | |
| 26 | | Cyclohexane | |
| 27 | | Isopropyl ether | |

### Example 7. Preparation of Crystal Form A of Compound of Formula 1

8 mg of the compound represented by formula 1 was dissolved in 0.3 mL of DMSO, and the solution was volatilized for crystallization. The product was identified as the crystal form A by X-ray powder diffraction.

The solids obtained using the solvents in Table 6 with reference to the above preparation method were identified as the crystal form A by X-ray powder diffraction.

**Table 6. The preparation of the crystal form A of the compound**

| NO. | Compound of formula 1 | Solvent |
|---|---|---|
| 1 | 8 mg | 0.6 mL of dichloromethane |
| 2 | | 0.08 mL of trichloromethane |
| 3 | | 0.44 mL of N,N-dimethylformamide |
| 4 | | 0.2 mL of N,N-dimethylacetamide |
| 5 | | 1.2 mL of DMSO/tetrahydrofuran (v/v = 1:5) |

### Example 8. Preparation of Crystal Form A of Compound of Formula 1

The crystal form A of the compound of formula 1 (12.30 g, 27.42 mmol) was dispersed in 120 mL of absolute ethanol and slurried and stirred at 75 °C for 1 h. The mixture was cooled to room temperature, stirred for 12 h, and filtered, and the filter cake was collected. The resulting solid was ground, then dispersed in 100 mL of absolute ethanol, and stirred and slurried for 1 h. The mixture was filtered, and the filter cake was collected, washed with ethanol (20 mL × 2), and dried *in vacuo* at 25 °C for 2 h to give the compound of formula 1 (8.96 g, yield: 72.8%). The product was identified as the crystal form A by X-ray powder diffraction.

### Example 9. Preparation of Crystal Form B of Compound of Formula 1

The crude compound of formula 1 (0.856 g, 1.91 mmol) was purified by high performance liquid chromatography (column: SharpSil-T Prep C18 150 × 30 mm, 5 µm; mobile phase A: water (containing 10 mmol/L ammonium bicarbonate); mobile phase B: acetonitrile; 10 min gradient: acetonitrile 32%; flow rate: 30 mL/min), and the resulting solution was lyophilized to give the compound of formula 1 (0.09 g, yield: 10.9%).

According to X-ray powder diffraction analysis, the product was defined as crystal form B. Its X-ray powder diffraction data are shown in Table 7, and its X-ray powder diffraction pattern is shown in FIG. 3.

Its DSC thermogram showed an endothermic peak at 258.01 °C. Its TGA thermogram showed that the compound had a weight loss of 0.69% from 40 °C to 160 °C.

**Table 7**

| Peak No. | 2θ value [° or degrees] | D[Å] | Relative intensity% |
|---|---|---|---|
| 1 | 5.910 | 14.94258 | 2.7 |
| 2 | 8.002 | 11.03983 | 100.0 |
| 3 | 8.523 | 10.36612 | 4.0 |
| 4 | 8.978 | 9.84145 | 5.8 |
| 5 | 9.248 | 9.55496 | 14.8 |
| 6 | 10.322 | 8.56283 | 2.9 |
| 7 | 11.985 | 7.37862 | 37.4 |
| 8 | 13.412 | 6.59669 | 50.4 |
| 9 | 15.984 | 5.54030 | 3.2 |
| 10 | 17.526 | 5.05617 | 12.2 |
| 11 | 17.929 | 4.94346 | 11.5 |
| 12 | 18.380 | 4.82305 | 2.8 |
| 13 | 18.964 | 4.67591 | 3.6 |
| 14 | 19.527 | 4.54238 | 2.1 |
| 15 | 21.090 | 4.20915 | 3.6 |
| 16 | 21.760 | 4.08095 | 3.3 |
| 17 | 22.444 | 3.95819 | 1.7 |
| 18 | 22.893 | 3.88155 | 4.3 |
| 19 | 24.545 | 3.62395 | 1.6 |
| 20 | 25.561 | 3.48208 | 1.4 |
| 21 | 26.974 | 3.30282 | 2.5 |
| 22 | 27.813 | 3.20512 | 1.5 |
| 23 | 31.982 | 2.79618 | 1.0 |
| 24 | 34.426 | 2.60306 | 1.0 |
| 25 | 39.063 | 2.30404 | 0.8 |

### Example 10. Preparation of Crystal Form a of Hydrochloride of Compound of Formula 1

120 mg of the compound represented by formula 1 was added to 9 mL of tetrahydrofuran/ethanol (v/v = 2:1) and dissolved by stirring at 60 °C. The solution was cooled to room temperature, and 145 µL of a 2 M solution of hydrochloric acid in ethanol was added. The mixture was stirred for crystallization and filtered, and the solid was dried *in vacuo* to give an off-white solid.

According to X-ray powder diffraction analysis, the product was defined as crystal form a of hydrochloride. Its XRPD pattern is shown in FIG. 4, and its characteristic peak positions are shown in Table 8. Its DSC thermogram showed an endothermic peak at 283.01 °C. Its TGA thermogram showed a weight loss of 1.53% from 30 °C to 170 °C and a weight loss of 6.49% from 170 °C to 258 °C. Its ion chromatography results showed a chloride ion content of 7.08%.

**Table 8**

| Peak No. | 2θ [° or degrees] | d [Å] | Relative intensity% |
|---|---|---|---|
| 1 | 8.164 | 10.82174 | 23.4 |
| 2 | 10.795 | 8.18890 | 30.5 |
| 3 | 11.375 | 7.77295 | 25.5 |
| 4 | 12.281 | 7.20122 | 54.9 |
| 5 | 13.481 | 6.56282 | 25.8 |
| 6 | 14.813 | 5.97552 | 23.5 |
| 7 | 15.561 | 5.68979 | 7.0 |
| 8 | 15.966 | 5.54658 | 6.4 |
| 9 | 16.296 | 5.43486 | 13.9 |
| 10 | 17.195 | 5.15281 | 72.6 |
| 11 | 17.606 | 5.03350 | 100.0 |
| 12 | 18.208 | 4.86842 | 18.1 |
| 13 | 19.659 | 4.51221 | 10.9 |
| 14 | 20.026 | 4.43024 | 20.4 |
| 15 | 21.523 | 4.12532 | 46.9 |
| 16 | 22.387 | 3.96815 | 8.9 |
| 17 | 22.982 | 3.86674 | 33.7 |
| 18 | 23.845 | 3.72872 | 47.4 |
| 19 | 24.814 | 3.58527 | 80.7 |
| 20 | 25.363 | 3.50882 | 20.0 |
| 21 | 25.962 | 3.42920 | 41.0 |
| 22 | 27.149 | 3.28192 | 19.5 |
| 23 | 28.223 | 3.15939 | 13.7 |
| 24 | 28.541 | 3.12499 | 5.5 |
| 25 | 29.207 | 3.05522 | 15.1 |
| 26 | 29.683 | 3.00731 | 11.4 |
| 27 | 31.414 | 2.84536 | 14.3 |
| 28 | 33.172 | 2.69852 | 2.0 |
| 29 | 34.694 | 2.58350 | 4.7 |
| 30 | 39.949 | 2.25497 | 1.6 |

### Example 11. Preparation of Crystal Form b of Hydrochloride of Compound of Formula 1

120 mg of the compound represented by formula 1 was dissolved in 1 mL of dichloromethane/methanol (v/v = 2:1), and 290 µL of a 2 M solution of hydrochloric acid in ethanol was added. The mixture was stirred for precipitation and filtered, and the solid was dried *in vacuo* to give an off-white solid.

According to X-ray powder diffraction analysis, the product was defined as crystal form b of hydrochloride. Its XRPD pattern is shown in FIG. 5, and its characteristic peak positions are shown in Table 9. Its DSC thermogram showed endothermic peaks at 49.81 °C, 258.00 °C, and 284.29 °C. Its TGA thermogram showed a weight loss of 1.88% from 30 °C to 150 °C and a weight loss of 8.12% from 150 °C to 235 °C. Its ion chromatography results showed a chloride ion content of 13.19%.

DVS testing showed that the sample had a hygroscopic weight gain of about 3.26% under normal storage conditions (i.e., 25 °C and 60% RH), a hygroscopic weight gain of about 3.65% under accelerated experimental conditions (i.e., 70% RH), and a hygroscopic weight gain of about 4.60% under extreme conditions (90% RH). In the process of the humidity changing from 0% to 95% RH, the desorption process of the sample substantially overlapped with its adsorption process. In addition, after DVS testing, the crystal form was re-identified, and the results showed that the crystal form did not change.

**Table 9**

| Peak No. | 2θ [° or degrees] | d [Å] | Relative intensity% |
|---|---|---|---|
| 1 | 5.166 | 17.09206 | 52.8 |
| 2 | 7.759 | 11.38483 | 68.4 |
| 3 | 10.741 | 8.23026 | 42.1 |
| 4 | 14.201 | 6.23152 | 20.4 |
| 5 | 14.887 | 5.94587 | 53.3 |
| 6 | 15.685 | 5.64527 | 45.8 |
| 7 | 16.604 | 5.33497 | 88.6 |
| 8 | 17.641 | 5.02360 | 44.9 |
| 9 | 18.963 | 4.67606 | 43.0 |
| 10 | 19.807 | 4.47884 | 2.1 |
| 11 | 20.433 | 4.34298 | 2.7 |
| 12 | 21.059 | 4.21524 | 15.9 |
| 13 | 21.855 | 4.06339 | 100.0 |
| 14 | 22.337 | 3.97680 | 60.5 |
| 15 | 22.949 | 3.87224 | 78.6 |
| 16 | 24.269 | 3.66444 | 29.3 |
| 17 | 25.838 | 3.44546 | 10.5 |
| 18 | 26.448 | 3.36724 | 82.8 |
| 19 | 27.540 | 3.23626 | 41.7 |
| 20 | 28.187 | 3.16337 | 29.9 |
| 21 | 28.606 | 3.11801 | 21.2 |
| 22 | 29.149 | 3.06115 | 40.5 |
| 23 | 30.188 | 2.95813 | 16.1 |
| 24 | 30.880 | 2.89339 | 5.0 |
| 25 | 31.539 | 2.83441 | 7.7 |
| 26 | 32.330 | 2.76685 | 16.3 |
| 27 | 33.121 | 2.70256 | 21.5 |
| 28 | 33.450 | 2.67668 | 19.2 |
| 29 | 33.780 | 2.65132 | 16.5 |
| 30 | 34.670 | 2.58528 | 13.1 |
| 31 | 35.625 | 2.51809 | 7.8 |
| 32 | 36.219 | 2.47819 | 1.5 |
| 34 | 37.702 | 2.38405 | 4.3 |
| 35 | 39.712 | 2.26787 | 3.6 |
| 36 | 40.206 | 2.24112 | 1.7 |
| 37 | 40.767 | 2.21161 | 4.5 |
| 38 | 41.722 | 2.16313 | 4.7 |
| 39 | 43.732 | 2.06825 | 6.0 |

### Example 12. Preparation of Crystal Form c of Hydrochloride of Compound of Formula 1

7 mg of the compound represented by formula 1 was added to 0.4 mL of 10% water/isopropanol and dissolved by stirring at 60 °C. The solution was cooled to room temperature, 8.5 µL of a 2 M solution of hydrochloric acid in ethanol was added, and 0.8 mL of isopropyl acetate was added. The mixture was stirred for crystallization and filtered, and the solid was dried *in vacuo* to give an off-white solid.

According to X-ray powder diffraction analysis, the product was defined as crystal form c of hydrochloride. Its XRPD pattern is shown in FIG. 6, and its characteristic peak positions are shown in Table 10. Its DSC thermogram showed endothermic peaks at 71.69 °C and 190.85 °C. Its TGA thermogram showed a weight loss of 16.01% from 30 °C to 180 °C.

**Table 10**

| Peak No. | 2θ [° or degrees] | d [Å] | Relative intensity% |
|---|---|---|---|
| 1 | 5.095 | 17.33093 | 76.1 |
| 2 | 8.779 | 10.06407 | 100.0 |
| 3 | 10.203 | 8.66238 | 65.1 |
| 4 | 25.834 | 3.44590 | 73.0 |

### Example 13. Preparation of Crystal Form α of Sulfate of Compound of Formula 1

7 mg of the compound represented by formula 1 was added to 0.4 mL of 10% water/isopropanol and dissolved by stirring at 60 °C. The solution was cooled to room temperature, and 8.5 µL of a 2 M solution of sulfuric acid in ethanol was added. The mixture was stirred for crystallization and centrifuged, and the solid was dried *in vacuo* to give an off-white solid.

According to X-ray powder diffraction analysis, the product was defined as crystal form α of sulfate. Its XRPD pattern is shown in FIG. 7, and its characteristic peak positions are shown in Table 11. Its DSC thermogram showed endothermic peaks at 62.16 °C, 196.17 °C, 249.16 °C, and 252.16 °C. Its TGA thermogram showed a weight loss of 9.75% from 30 °C to 165 °C.

**Table 11**

| Peak No. | 2θ [° or degrees] | d [Å] | Relative intensity% |
|---|---|---|---|
| 1 | 5.180 | 17.04661 | 100.0 |
| 2 | 8.955 | 9.86697 | 33.9 |
| 3 | 10.380 | 8.51568 | 16.2 |
| 4 | 13.767 | 6.42715 | 16.4 |
| 5 | 15.578 | 5.68392 | 14.3 |
| 6 | 18.063 | 4.90706 | 7.5 |
| 7 | 18.781 | 4.72110 | 5.9 |
| 8 | 25.809 | 3.44922 | 24.9 |

### Example 14. Preparation of Crystal Form α of Sulfate of Compound of Formula 1

7 mg of the compound represented by formula 1 was added to 0.6 mL of tetrahydrofuran/ethanol (v/v = 2:1) and dissolved by stirring at 60 °C. The solution was cooled to room temperature, and 8.5 µL of a 2 M solution of sulfuric acid in ethanol was added. The mixture was stirred for crystallization and centrifuged, and the solid was dried *in vacuo* to give an off-white solid. The product was identified as the crystal form α of sulfate by X-ray powder diffraction.

### Example 15. Preparation of Crystal Form α of Sulfate of Compound of Formula 1

7 mg of the compound represented by formula 1 was added to 0.1 mL of dichloromethane/methanol (v/v = 2:1)) and dissolved by stirring at 60 °C. The solution was cooled to room temperature, and 8.5 µL of a 2 M solution of sulfuric acid in ethanol was added. The mixture was stirred for crystallization and centrifuged, and the solid was dried *in vacuo* to give an off-white solid. The product was identified as the crystal form α of sulfate by X-ray powder diffraction.

### Example 16. Preparation of Crystal Form α of Phosphate of Compound of Formula 1

120 mg of the compound represented by formula 1 was added to 7 mL of 10% water/isopropanol and dissolved by stirring at 60 °C, and 145 µL of a 2 M solution of phosphoric acid in ethanol was added. The mixture was stirred at room temperature for crystallization and filtered, and the solid was dried *in vacuo* to give an off-white solid.

According to X-ray powder diffraction analysis, the product was defined as crystal form **α** of phosphate. Its XRPD pattern is shown in FIG. 8, and its characteristic peak positions are shown in Table 12. Its DSC thermogram showed endothermic peaks at 60.98 °C and 180.14 °C. Its TGA thermogram showed a weight loss of 4.67% from 30 °C to 170 °C. Its ion chromatography results showed a phosphate ion content of 18.47%.

DVS testing showed that the sample had a hygroscopic weight gain of about 11.72% under normal storage conditions (i.e., 25 °C and 60% RH), a hygroscopic weight gain of about 13.94% under accelerated experimental conditions (i.e., 70% RH), and a hygroscopic weight gain of about 23.17% under extreme conditions (90% RH). In the process of the humidity changing from 0% to 95% RH, the desorption process of the sample substantially overlapped with its adsorption process. In addition, after DVS testing, the crystal form was re-identified, and the results showed that the crystal form did not change.

**Table 12**

| Peak No. | 2θ [° or degrees] | d [Å] | Relative intensity% |
|---|---|---|---|
| 1 | 5.175 | 17.06419 | 100.0 |
| 2 | 9.006 | 9.81132 | 19.9 |
| 3 | 10.437 | 8.46905 | 13.8 |
| 4 | 11.000 | 8.03722 | 6.2 |
| 5 | 13.863 | 6.38265 | 17.6 |
| 6 | 15.707 | 5.63738 | 17.2 |
| 7 | 18.225 | 4.86384 | 8.4 |
| 8 | 18.979 | 4.67236 | 10.0 |
| 9 | 20.767 | 4.27380 | 2.0 |
| 10 | 25.682 | 3.46602 | 10.4 |
| 11 | 26.599 | 3.34851 | 1.2 |

### Example 17. Preparation of Crystal Form a of Phosphate of Compound of Formula 1

7 mg of the compound represented by formula 1 was added to 0.6 mL of tetrahydrofuran/ethanol (v/v = 2:1) and dissolved by stirring at 60 °C, and 8.5 µL of a 2 M solution of phosphoric acid in ethanol was added. The mixture was stirred at room temperature for crystallization and centrifuged, and the solid was dried *in vacuo* and identified as the crystal form α of phosphate by X-ray powder diffraction.

### Example 18. Preparation of Amorphous Phosphate of Compound of Formula 1

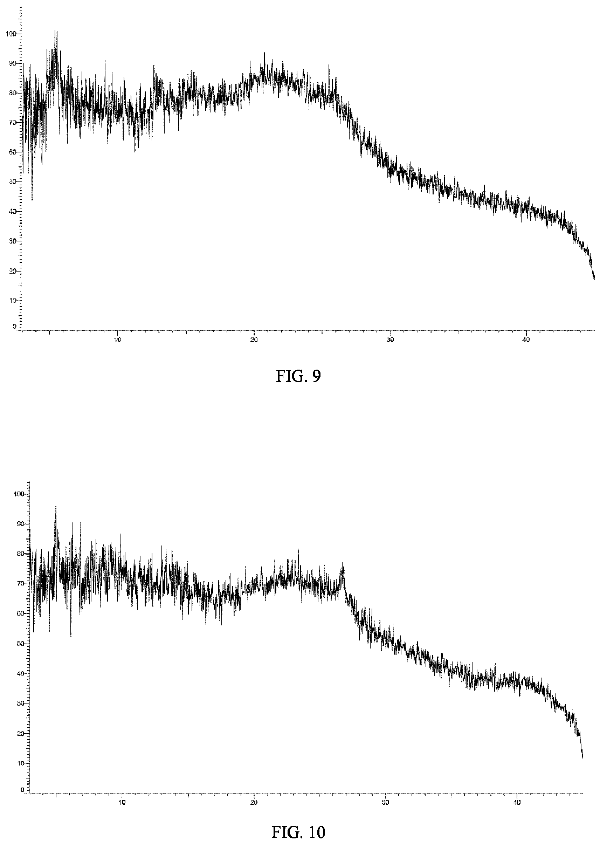

7 mg of the compound represented by formula 1 was dissolved in 0.1 mL of dichloromethane/methanol (v/v = 2:1), and 8.5 µL of a 2 M solution of phosphoric acid in ethanol was added. The mixture was stirred overnight, and 0.6 mL of MTBE was added. The mixture was stirred for crystallization and centrifuged, and the solid was dried *in vacuo* to give an off-white solid. The product was identified as an amorphous form by X-ray powder diffraction. Its XRPD pattern is shown in FIG. 9. Its ion chromatography results showed a phosphate ion content of 20.82%.

### Example 19. Preparation of Amorphous Mesylate of Compound of Formula 1

7 mg of the compound represented by formula 1 was added to 0.1 mL of dichloromethane/methanol (v/v = 2:1) and dissolved by stirring at room temperature, and 8.5 µL of a 2 M solution of methanesulfonic acid in ethanol was added. The mixture was stirred for crystallization and centrifuged, and the solid was dried *in vacuo* to give an off-white solid. The product was identified as an amorphous form by X-ray powder diffraction. Its XRPD pattern is shown in FIG. 10. Its ion chromatography results showed a mesylate ion content of 16.66%.

### Example 20. Preparation of Crystal Form I of Succinate of Compound of Formula 1

120 mg of the compound represented by formula 1 was dissolved in 0.6 mL of dichloromethane/methanol (v/v = 2:1), and 34 mg of solid succinic acid was added. The mixture was stirred for crystallization and filtered, and the solid was dried *in vacuo* to give an off-white solid.

According to X-ray powder diffraction analysis, the product was defined as crystal form I of succinate. Its XRPD pattern is shown in FIG. 11, and its characteristic peak positions are shown in Table 13. Its DSC thermogram showed endothermic peaks at 196.14 °C and 259.26 °C. Its TGA thermogram showed a weight loss of 1.35% from 30 °C to 150 °C and a weight loss of 20.52% from 150 °C to 230 °C. Its ion chromatography results showed a succinate ion content of 20.85%.

DVS testing showed that the sample had a hygroscopic weight gain of about 0.29% under normal storage conditions (i.e., 25 °C and 60% RH), a hygroscopic weight gain of about 0.38% under accelerated experimental conditions (i.e., 70% RH), and a hygroscopic weight gain of about 0.64% under extreme conditions (90% RH). In the process of the humidity changing from 0% to 95% RH, the desorption process of the sample substantially overlapped with its adsorption process. In addition, after DVS testing, the crystal form was re-identified, and the results showed that the crystal form did not change.

**Table 13**

| Peak No. | 2θ [° or degrees] | d [Å] | Relative intensity% |
|---|---|---|---|
| 1 | 6.106 | 14.46227 | 40.4 |
| 2 | 7.931 | 11.13816 | 3.3 |
| 3 | 8.589 | 10.28650 | 8.6 |
| 4 | 9.156 | 9.65047 | 5.9 |
| 5 | 10.134 | 8.72129 | 7.1 |
| 6 | 12.276 | 7.20434 | 100.0 |
| 7 | 14.812 | 5.97612 | 11.2 |
| 8 | 15.357 | 5.76528 | 3.1 |
| 9 | 17.517 | 5.05888 | 35.0 |
| 10 | 20.400 | 4.34985 | 25.8 |
| 11 | 21.650 | 4.10154 | 1.4 |
| 12 | 22.492 | 3.94981 | 0.9 |
| 13 | 23.228 | 3.82622 | 2.2 |
| 14 | 24.213 | 3.67279 | 4.5 |
| 15 | 24.836 | 3.58212 | 1.5 |
| 16 | 27.249 | 3.27007 | 2.1 |
| 17 | 28.110 | 3.17188 | 2.9 |
| 18 | 29.362 | 3.03938 | 3.7 |
| 19 | 31.061 | 2.87688 | 1.4 |
| 20 | 32.673 | 2.73858 | 1.7 |
| 21 | 35.236 | 2.54499 | 0.8 |
| 22 | 38.514 | 2.33563 | 3.4 |
| 23 | 41.059 | 2.19651 | 1.0 |
| 24 | 41.718 | 2.16332 | 1.4 |

### Example 21. Preparation of Crystal Form I of Fumarate of Compound of Formula 1

120 mg of the compound represented by formula 1 was added to 9 mL of tetrahydrofuran/ethanol (v/v = 2:1) and dissolved by stirring at 60 °C, and 34 mg of fumaric acid was added. The mixture was stirred at room temperature for crystallization and filtered, and the solid was dried *in vacuo* to give an off-white solid.

According to X-ray powder diffraction analysis, the product was defined as crystal form I of fumarate. Its XRPD pattern is shown in FIG. 12, and its characteristic peak positions are shown in Table 14. Its DSC thermogram showed an endothermic peak at 245.22 °C. Its TGA thermogram showed a weight loss of 0.19% from 30 °C to 165 °C and a weight loss of 20.27% from 165 °C to 270 °C. Its ion chromatography results showed a fumarate ion content of 20.76%.

DVS testing showed that the sample had a hygroscopic weight gain of about 0.21% under normal storage conditions (i.e., 25 °C and 60% RH), a hygroscopic weight gain of about 0.24% under accelerated experimental conditions (i.e., 70% RH), and a hygroscopic weight gain of about 0.38% under extreme conditions (90% RH). In the process of the humidity changing from 0% to 95% RH, the desorption process of the sample substantially overlapped with its adsorption process. In addition, after DVS testing, the crystal form was re-identified, and the results showed that the crystal form did not change.

**Table 14**

| Peak No. | 2θ [° or degrees] | d [Å] | Relative intensity% |
|---|---|---|---|
| 1 | 6.163 | 14.32979 | 42.9 |
| 2 | 7.982 | 11.06787 | 13.9 |
| 3 | 8.552 | 10.33095 | 39.9 |
| 4 | 9.201 | 9.60349 | 5.6 |
| 5 | 10.102 | 8.74903 | 18.4 |
| 6 | 11.251 | 7.85805 | 3.2 |
| 7 | 12.317 | 7.18055 | 100.0 |
| 8 | 14.750 | 6.00110 | 10.4 |
| 9 | 15.397 | 5.75029 | 1.4 |
| 10 | 16.001 | 5.53465 | 1.9 |
| 11 | 16.346 | 5.41860 | 1.8 |
| 12 | 17.407 | 5.09057 | 48.0 |
| 13 | 20.302 | 4.37062 | 21.4 |
| 14 | 21.069 | 4.21322 | 7.2 |
| 15 | 21.796 | 4.07426 | 4.0 |
| 16 | 22.678 | 3.91790 | 11.7 |
| 17 | 23.331 | 3.80956 | 15.3 |
| 18 | 24.336 | 3.65452 | 35.6 |
| 19 | 25.660 | 3.46884 | 1.7 |
| 20 | 26.692 | 3.33712 | 8.7 |
| 21 | 27.279 | 3.26662 | 15.1 |
| 22 | 28.148 | 3.16767 | 15.1 |
| 23 | 29.337 | 3.04197 | 2.3 |
| 24 | 30.836 | 2.89742 | 4.0 |
| 25 | 32.681 | 2.73795 | 3.4 |
| 26 | 33.718 | 2.65601 | 1.0 |
| 27 | 35.256 | 2.54363 | 0.7 |
| 28 | 38.215 | 2.35317 | 1.4 |
| 29 | 39.253 | 2.29332 | 0.7 |
| 30 | 41.329 | 2.18282 | 1.7 |

### Example 22. Preparation of Crystal Form I of Fumarate of Compound of Formula 1

7 mg of the compound represented by formula 1 was added to 0.4 mL of 10% water/isopropanol and dissolved by stirring at 60 °C, and 2 mg of fumaric acid was added. The mixture was stirred at room temperature for crystallization and filtered, and the solid was dried *in vacuo* and identified as the crystal form I by X-ray powder diffraction.

### Example 23. Preparation of Crystal Form I of Fumarate of Compound of Formula 1

7 mg of the compound represented by formula 1 was added to 0.6 mL of tetrahydrofuran/ethanol (v/v = 2: 1) and dissolved by stirring at 60 °C, and 2 mg of fumaric acid was added. The mixture was stirred at room temperature for crystallization and filtered, and the solid was dried *in vacuo* and identified as the crystal form I by X-ray powder diffraction.

### Example 24. Preparation of Crystal Form I of Fumarate of Compound of Formula 1

7 mg of the compound represented by formula 1 was added to 0.1 mL of dichloromethane/methanol (v/v = 2:1)) and dissolved by stirring at 60 °C, and 2 mg of fumaric acid was added. The mixture was stirred at room temperature for crystallization and filtered, and the solid was dried *in vacuo* and identified as the crystal form I by X-ray powder diffraction.

### Example 25. Preparation of Crystal Form a of Maleate of Compound of Formula 1

7 mg of the compound represented by formula 1 was dissolved in 0.1 mL of dichloromethane/methanol (v/v = 2:1), and 8.5 µL of a 2 M solution of maleic acid in ethanol was added. The mixture was stirred overnight at room temperature, and 0.6 mL of methyl tert-butyl ether was added. The mixture was stirred for crystallization and centrifuged, and the solid was dried *in vacuo* to give an off-white solid. According to X-ray powder diffraction analysis, the product was defined as crystal form a of maleate. Its XRPD pattern is shown in FIG. 13, and its characteristic peak positions are shown in Table 15. Its DSC thermogram showed endothermic peaks at 215.91 °C and 258.32 °C. Its TGA thermogram showed a weight loss of 2.49% from 30 °C to 130 °C and a weight loss of 9.95% from 130 °C to 260 °C. Its ion chromatography results showed a maleate ion content of 9.37%.

**Table 15**

| Peak No. | 2θ [° or degrees] | d [Å] | Relative intensity% |
|---|---|---|---|
| 1 | 7.902 | 11.17913 | 100.0 |
| 2 | 8.629 | 10.23924 | 24.2 |
| 3 | 9.109 | 9.70009 | 23.3 |
| 4 | 9.389 | 9.41225 | 32.4 |
| 5 | 11.879 | 7.44434 | 34.0 |
| 6 | 13.509 | 6.54940 | 12.0 |
| 7 | 14.285 | 6.19516 | 10.6 |
| 8 | 15.172 | 5.83481 | 6.2 |
| 9 | 15.683 | 5.64587 | 31.9 |
| 10 | 17.043 | 5.19846 | 16.7 |
| 11 | 17.453 | 5.07713 | 13.9 |
| 12 | 17.871 | 4.95931 | 26.2 |
| 13 | 19.572 | 4.53206 | 10.5 |
| 14 | 20.015 | 4.43260 | 16.4 |
| 15 | 20.792 | 4.26881 | 5.2 |
| 16 | 21.632 | 4.10479 | 31.4 |
| 17 | 22.862 | 3.88671 | 6.9 |
| 18 | 23.136 | 3.84129 | 5.4 |
| 19 | 24.489 | 3.63210 | 12.7 |
| 20 | 25.672 | 3.46734 | 18.9 |
| 21 | 26.559 | 3.35349 | 5.4 |
| 22 | 27.631 | 3.22576 | 9.9 |
| 23 | 28.888 | 3.08820 | 3.6 |
| 24 | 29.887 | 2.98725 | 1.0 |
| 25 | 30.404 | 2.93760 | 4.5 |
| 26 | 31.069 | 2.87618 | 3.6 |
| 27 | 31.919 | 2.80148 | 3.1 |

### Example 26. Preparation of Amorphous p-Toluenesulfonate of Compound of Formula 1

7 mg of the compound represented by formula 1 was dissolved in 0.1 mL of dichloromethane/methanol (v/v = 2:1), and 8.5 µL of a 2 M solution of p-toluenesulfonic acid in ethanol was added. The mixture was stirred overnight, and 0.6 mL of MTBE was added. The mixture was stirred for crystallization and centrifuged, and the solid was dried *in vacuo* to give an off-white solid. The product was identified as an amorphous form by X-ray powder diffraction. Its XRPD pattern is shown in FIG. 14. Its ion chromatography results showed a p-toluenesulfonate ion content of 26.91%.

### Example 27. Preparation of Amorphous L-Tartrate of Compound of Formula 1

7 mg of the compound represented by formula 1 was added to 0.4 mL of 10% water/isopropanol and dissolved by stirring at 60 °C, and 8.5 µL of a 2 M solution of tartaric acid in ethanol was added. The mixture was stirred overnight, and 0.8 mL of isopropyl acetate was added. The mixture was stirred for crystallization and centrifuged, and the solid was dried *in vacuo* to give an off-white solid. The product was identified as an amorphous form by X-ray powder diffraction. Its XRPD pattern is shown in FIG. 15. Its ion chromatography results showed an L-tartrate ion content of 31.43%.

### Example 28. Preparation of Crystal Form i of Hemifumarate of Compound of Formula 1

10.0 g of the compound of formula 1 and 1.294 g of fumaric acid were taken, and 800 mL of absolute methanol was added. The mixture was heated at reflux. After the solid dissolved, the solution was naturally cooled for crystallization to give the hemifumarate of the compound of formula 1. 0.5 g of the hemifumarate of the compound of formula 1 was taken and placed in a reaction flask, and 15 mL of absolute methanol was added. The mixture was refluxed, and the solid dissolved. The heating was stopped, and the mixture was cooled for crystallization. The mixture was filtered under reduced pressure, and the filter cake was blow-dried at 45 °C to give a white solid. According to X-ray powder diffraction analysis, the product was defined as crystal form i of hemifumarate. Its XRPD pattern is shown in FIG. 16, and its characteristic peak positions are shown in Table 16. Its HPLC results showed a fumarate ion content of 11.3% (based on the anhydrous compound).

**Table 16**

| Peak No. | 2θ [° or degrees] | d [Å] | Relative intensity% |
|---|---|---|---|
| 1 | 3.95 | 22.37 | 39.2 |
| 2 | 6.83 | 12.93 | 6.8 |
| 3 | 7.30 | 12.10 | 5.3 |
| 4 | 7.89 | 11.20 | 61.8 |
| 5 | 8.43 | 10.48 | 38.4 |
| 6 | 9.95 | 8.88 | 1.4 |
| 7 | 10.58 | 8.36 | 5.9 |
| 8 | 10.97 | 8.06 | 46.0 |
| 9 | 11.85 | 7.46 | 100.0 |
| 10 | 14.17 | 6.24 | 18.5 |
| 11 | 14.57 | 6.07 | 0.8 |
| 12 | 15.04 | 5.89 | 1.4 |
| 13 | 15.30 | 5.79 | 1.4 |
| 14 | 15.80 | 5.60 | 0.6 |
| 15 | 16.36 | 5.42 | 0.5 |
| 16 | 17.13 | 5.17 | 2.2 |
| 17 | 17.88 | 4.96 | 0.8 |
| 18 | 18.50 | 4.79 | 3.7 |
| 19 | 19.30 | 4.59 | 1.7 |
| 20 | 20.50 | 4.33 | 1.0 |
| 21 | 20.86 | 4.26 | 5.6 |
| 22 | 21.38 | 4.15 | 3.1 |
| 23 | 22.03 | 4.03 | 1.5 |
| 24 | 22.47 | 3.95 | 3.3 |
| 25 | 23.10 | 3.85 | 1.0 |
| 26 | 23.50 | 3.78 | 1.8 |
| 27 | 23.77 | 3.74 | 1.0 |
| 28 | 24.29 | 3.66 | 0.3 |
| 29 | 25.19 | 3.53 | 3.9 |
| 30 | 26.14 | 3.41 | 1.0 |
| 31 | 26.63 | 3.34 | 0.3 |
| 32 | 27.83 | 3.20 | 2.3 |
| 33 | 28.08 | 3.18 | 2.4 |
| 34 | 28.53 | 3.13 | 1.8 |
| 35 | 29.11 | 3.07 | 2.5 |
| 36 | 30.93 | 2.89 | 0.5 |
| 37 | 31.53 | 2.84 | 0.8 |
| 38 | 32.09 | 2.79 | 0.7 |
| 39 | 34.33 | 2.61 | 0.7 |
| 40 | 34.65 | 2.59 | 0.4 |
| 41 | 35.36 | 2.54 | 0.6 |
| 42 | 35.81 | 2.51 | 0.4 |
| 43 | 36.03 | 2.49 | 0.5 |
| 44 | 38.19 | 2.35 | 0.8 |
| 45 | 38.47 | 2.34 | 0.5 |

### Example 29. Preparation of Crystal Form ii of Hemifumarate of Compound of Formula 1

5 mg of the crystal form i of hemifumarate of the compound shown in Example 28 was heated to 170 °C to give a product.

According to X-ray powder diffraction analysis, the product was defined as crystal form ii of hemifumarate. Its XRPD pattern is shown in FIG. 17, and its characteristic peak positions are shown in Table 17. Its DSC thermogram showed an endothermic peak at 235.42 °C. Its TGA thermogram showed a weight loss of 0.77% from 30 °C to 120 °C and a weight loss of 11.04% from 120 °C to 250 °C. Its ion chromatography results showed a fumarate ion content of 11.5%.

**Table 17**

| Peak No. | 2θ value [° or degrees] | d[Å] | Relative intensity% |
|---|---|---|---|
| 1 | 8.581 | 10.29616 | 27.0 |
| 2 | 9.892 | 8.93439 | 100.0 |
| 3 | 12.605 | 7.01689 | 4.9 |
| 4 | 14.336 | 6.17326 | 5.1 |
| 5 | 15.293 | 5.78903 | 3.5 |
| 6 | 17.287 | 5.12541 | 10.6 |
| 7 | 18.293 | 4.84595 | 2.5 |
| 8 | 19.342 | 4.58547 | 1.4 |
| 9 | 20.078 | 4.41894 | 7.8 |
| 10 | 22.996 | 3.86443 | 2.7 |
| 11 | 25.804 | 3.44989 | 1.1 |

### Example 30. Preparation of Crystal Form iii of Hemifumarate of Compound of Formula 1

500 mg of the free-state compound represented by formula 1 was added to 10 mL of methanol, and 1.2 mL of a 0.5 M solution of fumaric acid in methanol was added. The mixture was stirred for crystallization and centrifuged, and the solid was collected and dried *in vacuo* at 60 °C to give a product.

According to X-ray powder diffraction analysis, the product was defined as crystal form iii of hemifumarate. Its XRPD pattern is shown in FIG. 18, and its characteristic peak positions are shown in Table 18. Its DSC thermogram showed an endothermic peak at 235.45 °C. Its TGA thermogram showed a weight loss of 0.55% from 30 °C to 100 °C and a weight loss of 10.25% from 100 °C to 250 °C. Its ion chromatography results showed a fumarate ion content of 11.8%.

**Table 18**

| Peak No. | 2θ value [° or degrees] | d[Å] | Relative intensity% |
|---|---|---|---|
| 1 | 8.363 | 10.56470 | 100.0 |
| 2 | 11.005 | 8.03358 | 30.5 |
| 3 | 12.498 | 7.07667 | 16.6 |
| 4 | 14.267 | 6.20319 | 3.2 |
| 5 | 28.341 | 3.14651 | 0.9 |

### Experimental Example 1. Crystal Form Stability Study

Samples of the free-state crystal form A, the crystal form a of hydrochloride, the crystal form α of phosphate, the crystal form I of succinate, and the crystal form I of fumarate were taken, spread in open containers, and left to stand under conditions of light exposure (4500 Lux), high temperatures (40 °C and 60 °C), and high humidities (RH 75% and RH 92.5%) for 30 days to study their stability.

**Table 19**

| Conditions | Time (days) | Free-state crystal form A | | |
|---|---|---|---|---|
| | | Color and state | Purity% | Crystal form |
| Initial | 0 | Off-white solid | 99.4 | Crystal form A |
| 40 °C | 5 | Off-white solid | 99.2 | Did not change |
| | 10 | Off-white solid | 99.1 | Did not change |
| | 30 | Off-white solid | 98.6 | Did not change |
| 60 °C | 5 | Off-white solid | 99.0 | Did not change |
| | 10 | Off-white solid | 98.7 | Did not change |
| | 30 | Off-white solid | 97.5 | Did not change |
| 75% RH | 5 | Off-white solid | 99.4 | Did not change |
| | 10 | Off-white solid | 99.4 | Did not change |
| | 30 | Off-white solid | 99.4 | Did not change |
| 92.5% RH | 5 | Off-white solid | 99.4 | Did not change |
| | 10 | Off-white solid | 99.4 | Did not change |
| | 30 | Off-white solid | 99.4 | Did not change |
| Light exposure | 5 | Off-white solid | 96.3 | Did not change |
| | 10 | Off-white solid | 95.3 | Did not change |

**Table 18**

| Conditions | Time (days) | Crystal form a of hydrochloride | | |
|---|---|---|---|---|
| | | Color and state | Purity% | Crystal form |
| Initial | 0 | Off-white solid | 99.7 | Crystal form a of hydrochloride |
| 40 °C | 5 | Off-white solid | 99.7 | Did not change |
| | 10 | Off-white solid | 99.6 | Did not change |
| | 30 | Off-white solid | 99.5 | Did not change |
| 60 °C | 5 | Off-white solid | 99.6 | Did not change |
| | 10 | Off-white solid | 99.4 | Did not change |
| | 30 | Off-white solid | 98.8 | Did not change |
| 75% RH | 5 | Off-white solid | 99.7 | Did not change |
| | 10 | Off-white solid | 99.7 | Did not change |
| | 30 | Off-white solid | 99.7 | Did not change |
| 92.5% RH | 5 | Off-white solid | 99.7 | Changed |
| | 10 | Off-white solid | 99.7 | Changed |
| | 30 | Off-white solid | 99.7 | Changed |
| 4500 Lux | 5 | Off-white solid | 99.7 | Did not change |
| | 10 | Off-white solid | 99.2 | Did not change |
| | 30 | Off-white solid | 98.4 | Did not change |

| Conditions | Time (days) | Crystal form α of phosphate | | |
|---|---|---|---|---|
| | | Color and state | Purity% | Crystal form |
| Initial | 0 | Off-white solid | 99.7 | Crystal form α of phosphate |
| 40 °C | 5 | Off-white solid | 99.1 | Did not change |
| | 10 | Off-white solid | 98.8 | Did not change |
| | 30 | Off-white solid | 97.6 | Did not change |
| 60 °C | 5 | Off-white solid | 97.6 | Did not change |
| | 10 | Off-white solid | 96.5 | Did not change |
| | 30 | Off-white solid | 93.6 | Did not change |
| 75% RH | 5 | Off-white solid | 99.6 | Did not change |
| | 10 | Off-white solid | 99.6 | Did not change |
| | 30 | Off-white solid | 99.7 | Did not change |
| 92.5% RH | 5 | Off-white solid | 99.6 | Did not change |
| | 10 | Off-white solid | 99.6 | Did not change |
| | 30 | Off-white solid | 99.7 | Did not change |

| Conditions | Time (days) | Crystal form I of succinate | | |
|---|---|---|---|---|
| | | Color and state | Purity% | Crystal form |
| Initial | 0 | Off-white solid | 99.5 | Crystal form I of succinate |
| 40 °C | 7 | Off-white solid | 99.5 | Did not change |
| | 14 | Off-white solid | 99.4 | Did not change |
| | 30 | Off-white solid | 99.4 | Did not change |
| 60 °C | 7 | Off-white solid | 99.4 | Did not change |
| | 14 | Off-white solid | 99.4 | Did not change |
| | 30 | Off-white solid | 99.2 | Did not change |
| 75% RH | 7 | Off-white solid | 99.5 | Did not change |
| | 14 | Off-white solid | 99.5 | Did not change |
| | 30 | Off-white solid | 99.5 | Did not change |
| 92.5% RH | 7 | Off-white solid | 99.5 | Did not change |
| | 14 | Off-white solid | 99.5 | Did not change |
| | 30 | Off-white solid | 99.5 | Did not change |
| 4500 Lux | 7 | Off-white solid | 98.7 | Did not change |
| | 14 | Off-white solid | 97.7 | Did not change |
| | 30 | Off-white solid | 96.5 | Did not change |

| Conditions | Time (days) | Crystal form I of fumarate | | |
|---|---|---|---|---|
| | | Color and state | Purity% | Crystal form |
| Initial | 0 | Off-white solid | 99.8 | Crystal form I of fumarate |
| 40 °C | 5 | Off-white solid | 99.8 | Did not change |
| | 10 | Off-white solid | 99.7 | Did not change |
| | 30 | Off-white solid | 99.7 | Did not change |
| 60 °C | 5 | Off-white solid | 99.8 | Did not change |
| | 10 | Off-white solid | 99.7 | Did not change |
| | 30 | Off-white solid | 99.6 | Did not change |
| 75% RH | 5 | Off-white solid | 99.8 | Did not change |
| | 10 | Off-white solid | 99.8 | Did not change |
| | 30 | Off-white solid | 99.8 | Did not change |
| 92.5% RH | 5 | Off-white solid | 99.8 | Did not change |
| | 10 | Off-white solid | 99.8 | Did not change |
| | 30 | Off-white solid | 99.8 | Did not change |
| 4500 Lux | 5 | Off-white solid | 99.4 | Did not change |
| | 10 | Off-white solid | 99.0 | Did not change |
| | 30 | Off-white solid | 97.0 | Did not change |

| | | | | |
|---|---|---|---|---|
| Conclusion: The influencing-factor experiments show that: standing under conditions of a high temperature of 40 °C and high humidities of 75% and 92.5% for 30 days, the free-state crystal form A exhibited good physical and chemical stability; standing under conditions of high temperatures of 40 °C and 60 °C and a high humidity of 75% for 30 days, the crystal form a of hydrochloride exhibited good physical and chemical stability; standing under conditions of high humidities of 75% and 92.5% for 30 days, the crystal form α of phosphate exhibited good physical and chemical stability; standing under conditions of high temperatures of 40 °C and 60 °C and high humidities of 75% and 92.5% for 30 days, the crystal form I of succinate exhibited good physical and chemical stability; standing under conditions of high temperatures of 40 °C and 60 °C and high humidities of 75% and 92.5% for 30 days, the crystal form I of fumarate exhibited good physical and chemical stability. | | | | |

### Experimental Example 2. Long-Term/Accelerated Stability

The free-state crystal form A, the crystal form a of hydrochloride, the crystal form b of hydrochloride, the crystal form α of phosphate, the crystal form I of succinate, and the crystal form I of fumarate were left to stand under conditions of 25 °C/60% RH and 40 °C/75% RH to study their stability.

**Table 20**

| Sample | Standing conditions | Purity% | Purity% | Purity% | Purity% | Purity% | Crystal form |
|---|---|---|---|---|---|---|---|
| | | Initial | 1 month | 2 months | 3 months | 6 months | |
| Crystal form A | 25 °C/60% RH | 99.4 | 99.4 | 99.4 | 99.4 | 99.4 | A |
| | 40 °C/75% RH | 99.4 | 99.4 | 99.3 | 99.1 | 98.8 | A |

**Table 21**

| Sample | Standing conditions | Purity% | Purity% | Purity% | Purity% | Purity% | Crystal form |
|---|---|---|---|---|---|---|---|
| | | Initial | 1 month | 2 months | 3 months | 6 months | |
| Crystal form a of hydrochloride | 25 °C/60% RH | 99.7 | 99.7 | 99.7 | 99.7 | 99.7 | Did not change |
| | 40 °C/75% RH | 99.7 | 99.7 | 99.7 | 99.7 | 99.7 | Did not change |

| Sample | Standing conditions | Purity% | Purity% | Purity% | Purity% | Purity% | Crystal form |
|---|---|---|---|---|---|---|---|
| | | Initial | 1 month | 2 months | 3 months | 6 months | |
| Crystal form b of hydrochloride | 25 °C/60% RH | 99.7 | 99.7 | 99.7 | 99.7 | 99.7 | Did not change |
| | 40 °C/75% RH | 99.7 | 99.7 | 99.7 | 99.7 | 99.7 | Did not change |

| Sample | Standing conditions | Purity% | Purity% | Purity% | Purity% | Purity% | Crystal form |
|---|---|---|---|---|---|---|---|
| | | Initial | 1 month | 2 months | 3 months | 6 months | |
| Crystal form a of phosphate | 25 °C/60% RH | 99.7 | 99.6 | 99.6 | 99.7 | 99.6 | Did not change |
| | 40 °C/75% RH | 99.7 | 99.6 | 99.6 | 99.6 | 99.6 | Did not change |

| Sample | Standing conditions | Purity% | Purity% | Purity% | Purity% | Purity% | Crystal form |
|---|---|---|---|---|---|---|---|
| | | Initial | 1 month | 2 months | 3 months | 6 months | |
| Crystal form I of succinate | 25 °C/60% RH | 99.5 | 99.5 | 99.5 | 99.5 | 99.5 | Did not change |
| | 40 °C/75% RH | 99.5 | 99.5 | 99.5 | 99.5 | 99.5 | Did not change |

| Sample | Standing conditions | Purity% | Purity% | Purity% | Purity% | Purity% | Crystal form |
|---|---|---|---|---|---|---|---|
| | | Initial | 1 month | 2 months | 3 months | 6 months | |
| Crystal form I of fumarate | 25 °C/60% RH | 99.8 | 99.8 | 99.8 | 99.8 | 99.8 | Did not change |
| | 40 °C/75% RH | 99.8 | 99.8 | 99.8 | 99.8 | 99.8 | Did not change |

| | | | | | | | |
|---|---|---|---|---|---|---|---|
| Conclusion: The long-term and accelerated experiments show that: standing under conditions of 25 °C/60 RH and 40 °C/75 RH for 6 months, the crystal form A, the crystal form a of hydrochloride, the crystal form b of hydrochloride, the crystal form α of phosphate, the crystal form I of fumarate, and the crystal form I of succinate exhibited good physical and chemical stability. | | | | | | | |

## Claims

1. A crystal form A of (R)-3-((7-ethyl-6-oxo-5,6-dihydro-1,5-naphthyridin-3-yl)methyl)-*N*-methyl-1,2,3,4,4*a*,5-hexahydro-7*H*-pyrazino[2,1-*c*]pyrido[3,2-*e*][1,4]oxazepine-9-carboxamide, wherein an X-ray powder diffraction pattern expressed in terms of 2*θ* angles, which are diffraction angles, of the crystal form has characteristic peaks at 7.877, 11.871, and 17.876, preferably at 7.877, 9.115, 11.871, and 17.876; most preferably, the X-ray powder diffraction pattern expressed in terms of 2*θ* angles, which are diffraction angles, is shown in FIG. 2.

2. A pharmaceutically acceptable salt of (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, wherein the pharmaceutically acceptable salt is selected from the group consisting of hydrochloride, sulfate, phosphate, mesylate, succinate, fumarate, maleate, p-toluenesulfonate, L-tartrate, D-malate, L-malate, and citrate.

3. The pharmaceutically acceptable salt according to claim 2, wherein a chemical ratio of (*R*)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide to acid is 3:1-1:3, preferably 2:1-1:2, and more preferably 2:1, 1:1, or 1:2.

4. A preparation method for a pharmaceutically acceptable salt of (*R*)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, comprising a step of reacting (*R*)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide with an acid, wherein the acid is selected from the group consisting of hydrochloric acid, sulfuric acid, phosphoric acid, methanesulfonic acid, succinic acid, fumaric acid, maleic acid, p-toluenesulfonic acid, L-tartaric acid, D-malic acid, L-malic acid, and citric acid.

5. A fumarate of (*R*)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, wherein a chemical ratio of (*R*)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide to fumaric acid is 1:1.

6. A crystal form I of fumarate of (*R*)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, wherein an X-ray powder diffraction pattern expressed in terms of 2*θ* angles, which are diffraction angles, of the crystal form has characteristic peaks at 6.163, 8.552, 12.317, 17.407, and 24.336, preferably at 6.163, 8.552, 10.102, 12.317, 17.407, 20.302, 23.331, 24.336, 27.279, and 28.148, and more preferably at 6.163, 7.982, 8.552, 10.102, 12.317, 14.750, 17.407, 20.302, 21.069, 22.678, 23.331, 24.336, 26.692, 27.279, and 28.148; most preferably, the X-ray powder diffraction pattern expressed in terms of 2*θ* angles, which are diffraction angles, is shown in FIG. 12.

7. A succinate of (*R*)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, wherein a chemical ratio of (*R*)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide to succinic acid is 1:1.

8. A crystal form I of succinate of (*R*)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, wherein an X-ray powder diffraction pattern expressed in terms of 2*θ* angles, which are diffraction angles, of the crystal form has characteristic peaks at 6.106, 8.589, 12.276, 14.812, 17.517, and 20.400, preferably at 6.106, 8.589, 9.156, 10.134, 12.276, 14.812, 17.517, 20.400, and 24.213, and more preferably at 6.106, 7.931, 8.589, 9.156, 10.134, 12.276, 14.812, 15.357, 17.517, 20.400, 24.213, 29.362, and 38.514; most preferably, the X-ray powder diffraction pattern expressed in terms of 2*θ* angles, which are diffraction angles, is shown in FIG. 11.

9. A crystal form of (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide, wherein 2*θ* angles have a margin of error of ±0.2.

10. A pharmaceutical composition, comprising the following components:
i) the crystal form A of (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide according to claim 1, or the pharmaceutically acceptable salt of (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide according to any one of claims 2, 3, 5, and 7, or the crystal form according to claim 6 or 8; and
ii) one or more pharmaceutically acceptable excipients.

11. A method for preparing a pharmaceutical composition, comprising a step of mixing the crystal form A of (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide according to claim 1, or the pharmaceutically acceptable salt of (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide according to any one of claims 2, 3, 5, and 7, or the crystal form according to claim 6 or 8, with a pharmaceutically acceptable excipient.

12. Use of the crystal form A of (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide according to claim 1, or the pharmaceutically acceptable salt of (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide according to any one of claims 2, 3, 5, and 7, or the crystal form according to claim 6 or 8, or the composition according to claim 10, in the preparation of a PARP1 inhibitor.

13. Use of the crystal form A of (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e] [1,4]oxazepine-9-carboxamide according to claim 1, or the pharmaceutically acceptable salt of (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide according to any one of claims 2, 3, 5, and 7, or the crystal form according to claim 6 or 8, or the composition according to claim 10, in the preparation of a medicament for treating and/or preventing a cancer.

14. The use according to claim 13, wherein the cancer is selected from the group consisting of breast cancer, ovarian cancer, pancreatic cancer, prostate cancer, gastric cancer, colorectal cancer, lung cancer, renal cancer, hepatic cancer, cervical cancer, endometrial cancer, myeloma, leukemia, lymphoma, acoustic neuroma, basal cell carcinoma, cholangiocarcinoma, bladder cancer, brain cancer, bronchogenic carcinoma, sarcoma, chordoma, choriocarcinoma, craniopharyngioma, cystadenocarcinoma, embryonal carcinoma, hemangioendothelioma, ependymoma, epithelial cancer, esophageal cancer, essential thrombocytosis, Ewing sarcoma, testicular cancer, glioma, heavy chain disease, hemangioblastoma, medullary carcinoma, medulloblastoma, melanoma, meningioma, mesothelioma, neuroblastoma, NUT midline carcinoma, neuroglioma, bone cancer, nasopharyngeal carcinoma, oral cancer, thyroid cancer, pinealoma, polycythemia vera, retinoblastoma, sebaceous carcinoma, seminoma, skin cancer, squamous cell carcinoma, synovioma, sweat gland carcinoma, Waldenström macroglobulinemia, and Wilms tumor; preferably, the cancer is selected from the group consisting of breast cancer, ovarian cancer, pancreatic cancer, prostate cancer, gastric cancer, colorectal cancer, and lung cancer.
